# EUROPEAN PATENT SPECIFICATION

(11) **EP 0 699 069 B1**
(45) Date of publication and mention of the grant of the patent: **13.02.2002**
(21) Application number: 94915903.2
(22) Date of filing: 26.04.1994
(51) Int. Cl.: A61K 31/075, A61K 31/165, A61K 31/185, A61K 31/18, A61K 31/19, A61K 31/21, A61K 31/335, A61K 31/34, A61K 31/35, A61K 31/36, C07D 317/60, C07D 405/12

(54) **ENDOTHELIN RECEPTOR ANTAGONISTS**
ENDOTHELIN-REZEPTOR-ANTAGONISTEN
ANTAGONISTES DES RECEPTEURS DE L'ENDOTHELINE

(30) Priority: 27.04.1993 US 66818
(43) Date of publication of application: 06.03.1996
(73) Proprietor: SMITHKLINE BEECHAM CORPORATION, Philadelphia Pennsylvania 19101 (US)
(72) Inventor: COUSINS, Russell Donovan, Oxford, PA 19363 (US); ELLIOTT, John Duncan, Wayne, PA 19087 (US); LAGO, Maria Amparo, Audubon, PA 19403 (US); LEBER, Jack Dale, Audubon, PA 19403 (US); PEISHOFF, Catherine Elizabeth, West Chester, PA 19380 (US)
(74) Representative: Florence, Julia Anne
(86) International application number: US9404603
(87) International publication number: WO9425013

(56) References cited:
- WO-A-93/08799
- JOURNAL OF ORGANIC CHEMISTRY, vol. 25, no. 12, 23 January 1961 EASTON US, pages 2088-2091, C. F. KOELSCH 'Electrophilic Properties of Ethyl 3-Phenylindone-2-carboxylate'
- CHEMICAL ABSTRACTS, vol. 88, no. 25, 19 June 1978 Columbus, Ohio, US; abstract no. 190677p, page 751; column 2; & KONF. KHIM. ATSETILENA, 5TH, 1975 TIFLIS, USSR, pages 374-375, M. I. KOMENDANTOW ET AL. '1,3-Dipolar cycloaddition od diphenyldiazomethane to methyl and ethyl esters of phenylpropiolic acid and its nitrile'
- Bulletin of the Chemical Society of Japan, Volume 59, issued November 1986, (Tokyo, Japan), KIMIAKI YAMAMURA et al., "Formation of 2-Substituted 1,3-Diphenylindenes by an N-Bromosuccinimide Prompted Dehrocyclization of 2-Substituted 1,3,3-Triphenyl-1-Propenes", pages 3699-3701, see entire document.

## Description

### FIELD OF INVENTION

The present invention relates to novel indane and indene derivatives, pharmaceutical compositions containing these compounds and their use as endothelin receptor antagonists.

Endothelin (ET) is a highly potent vasoconstrictor peptide synthesized and released by the vascular endothelium. Endothelin exists as three isoforms, ET-1, ET-2 and ET-3. [Unless otherwise stated "endothelin" shall mean any or all of the isoforms of endothelin]. Endothelin has profound effects on the cardiovascular system, and in particular, the coronary, renal and cerebral circulation. Elevated or abnormal release of endothelin is associated with smooth muscle contraction which is involved in the pathogenesis of cardiovascular, cerebrovascular, respiratory and renal pathophysiology. Elevated levels of endothelin have been reported in plasma from patients with essential hypertension, acute myocardial infarction, subarachnoid hemorrhage, atherosclerosis, and patients with uraemia undergoing dialysis.

In vivo, endothelin has pronounced effects on blood pressure and cardiac output. An intravenous bolus injection of ET (0.1 to 3 nmol/kg) in rats causes a transient, dose-related depressor response (lasting 0.5 to 2 minutes) followed by a sustained, dose-dependent rise in arterial blood pressure which can remain elevated for 2 to 3 hours following dosing. Doses above 3 nmol/kg in a rat often prove fatal.

Endothelin appears to produce a preferential effect in the renal vascular bed. It produces a marked, long-lasting decrease in renal blood flow, accompanied by a significant decrease in GFR, urine volume, urinary sodium and potassium excretion. Endothelin produces a sustained antinatriuretic effect, despite significant elevations in atrial natriuretic peptide. Endothelin also stimulates plasma renin activity. These findings suggest that ET is involved in the regulation of renal function and is involved in a variety of renal disorders including acute renal failure, cyclosporine nephrotoxicity, radio contrast induced renal failure and chronic renal failure.

Studies have shown that in vivo, the cerebral vasculature is highly sensitive to both the vasodilator and vasoconstrictor effects of endothelin. Therefore, ET may be an important mediator of cerebral vasospasm, a frequent and often fatal consequence of subarachnoid hemorrhage.

ET also exhibits direct central nervous system effects such as severe apnea and ischemic lesions which suggests that ET may contribute to the development of cerebral infarcts and neuronal death.

ET has also been implicated in myocardial ischemia (Nichols et al. Br. J. Pharm. 99: 597-601, 1989 and Clozel and Clozel, Circ. Res., 65: 1193- 1200, 1989) coronary vasospasm (Fukuda et al., Eur. J. Pharm. 165: 301-304, 1989 and Lüscher, Circ. 83: 701, 1991) heart failure, proliferation of vascular smooth muscle cells, (Takagi, Biochem & Biophys, Res. Commun.; 168: 537-543, 1990, Bobek et al., Am. J. Phvsiol. 258:408-C415, 1990) and atherosclerosis, (Nakaki et al., Biochem. & Biophys. Res. Commun. 158: 880-881, 1989, and Lerman et al., New Eng. J. of Med. 325: 997-1001, 1991). Increased levels of endothelin have been shown after coronary balloon angioplasty (Kadel et al., No. 2491 Circ. 82: 627, 1990).

Further, endothelin has been found to be a potent constrictor of isolated mammalian airway tissue including human bronchus (Uchida et al., Eur J. of Pharm. 154: 227-228 1988, LaGente, Clin. Exp. Allergy 20: 343-348, 1990; and Springall et al., Lancet, 337: 697-701, 1991). Endothelin may play a role in the pathogenesis of interstitial pulmonary fibrosis and associated pulmonary hypertension, Glard et al., Third International Conference on Endothelin, 1993, p. 34 and ARDS (Adult Respiratory Distress Syndrome), Sanai et al., Supra, p. 112.

Endothelin has been associated with the induction of hemorrhagic and necrotic damage in the gastric mucosa (Whittle et al., Br. J. Pharm. 95: 1011-1013, 1988); Raynaud's phenomenon, Cinniniello et al., Lancet 337: 114-115, 1991); Crohn's Disease and ulcerative colitis, Munch et al., Lancet, Vol. 339, p. 381; Migraine (Edmeads, Headache, Feb. 1991 p 127); Sepsis (Weitzberg et al., Circ. Shock 33: 222-227, 1991; Pittet et al., Ann. Surg. 213: 262-264, 1991), Cyclosporin-induced renal failure or hypertension (Eur. J. Pharmacol., 180: 191-192, 1990, Kidney Int, 37: 1487-1491, 1990) and endotoxin shock and other endotoxin induced diseases (Biochem. Biophys. Res. Commun., 161: 1220-1227, 1989, Acta Physiol. Scand. 137: 317-318, 1989) and inflammatory skin diseases. (Clin Res. 41:451 and 484, 1993).

Endothelin has also been implicated in preclampsia of pregnancy. Clark et al., Am. J. Obstet. Gynecol. March 1992, p. 962-968; Kamor et al., N. Eng. J. of Med., Nov 22, 1990, p. 1486-1487; Dekker et al., Eur J. Ob. and Gyn. and Rep. Bio. 40 (1991) 215-220; Schiff et al., Am. J. Ostet. Gynecol. Feb 1992, p. 624-628; diabetes mellitus, Takahashi et al., Diabetologia (1990) 33:306-310; and acute vascular rejection following kidney transplant, Watschinger et al., Transplantation Vol. 52, No. 4, pp. 743-746.

Endothelin stimulates both bone resorption and anabolism and may have a role in the coupling of bone remodeling. Tatrai et al. Endocrinology, Vol. 131, p. 603-607.

Endothelin has been reported to stimulate the transport of sperm in the uterine cavity, Casey et al., J. Clin. Endo and Metabolism, Vol. 74, No. 1, p. 223-225, therefore endothelin antagonists may be useful as male contraceptives. Endothelin modulates the ovarian/menstrual cycle, Kenegsberg, J. of Clin. Endo. and Met., Vol. 74, No. 1, p. 12, and may also play a role in the regulation of penile vascular tone in man, Lau et al., Asia Pacific J. of Pharm., 1991, 6:287-292 and Tejada et al., J. Amer. Physio. Soc. 1991, H1078-H1085. Endothelin also mediates a potent contraction of human prostatic smooth muscle, Langenstroer et al., J. Urology, Vol. 149, p. 495-499.

Thus, endothelin receptor antagonists would offer a unique approach toward the pharmacotherapy of hypertension, renal failure, ischemia induced renal failure, sepsis-endotoxin induced renal failure, prophylaxis and/or treatment of radio-contrast induced renal failure, acute and chronic cyclosporin induced renal failure, cerebrovascular disease, myocardial ischemia, angina, heart failure, asthma, atherosclerosis, Raynaud's phenomenon, ulcers, sepsis, migraine, glaucoma, endotoxin shock, endotoxin induced multiple organ failure or disseminated intravascular coagulation, cyclosporin-induced renal failure and as an adjunct in angioplasty for prevention of restenosis, diabetes, preclampsia of pregnancy, bone remodeling, kidney transplant, male contraceptives, infertility and priaprism and benign prostatic hypertrophy.

### SUMMARY OF THE INVENTION

This invention comprises indane and indene derivatives represented by Formula (I) and pharmaceutical compositions containing these compounds, and their use as endothelin receptor antagonists which are useful in the treatment of a variety of cardiovascular and renal diseases including but not limited to:
stroke, cerebrovascular vasospasm, myocardial ischemia, angina, heart failure, atherosclerosis, and as an adjunct in angioplasty for prevention of restenosis.

### DETAILED DESCRIPTION OF THE INVENTION

The compounds of this invention are represented by structural Formula (I): 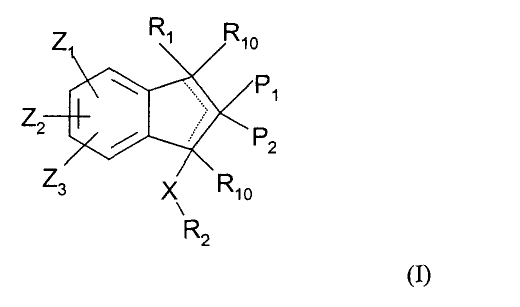 wherein:
R₁ is -X(CH₂)ₙAr or -X(CH₂)ₙR₈ or 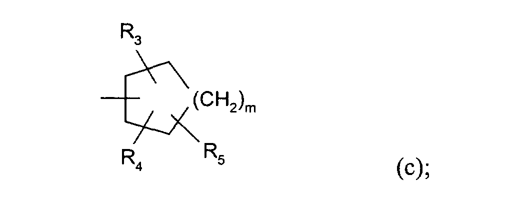
R₂ is hydrogen, Ar, C₁₋₄alkyl or (c);
P₁ is -X(CH₂)ₙR₈;
P₂ is -X(CH₂)ₙR₈ or -X-R₉-Y;
R₃ and R₅ are independently hydrogen, R₁₁, OH, C₁₋₈alkoxy, S(O)_{q}R₁₁, N(R₆)₂, Br, F, I, Cl, CF₃, NHCOR₆, R₁₁CO₂R₇, -X-R₉-Y or -X(CH₂)ₙR₈ wherein each methylene group within -X(CH₂)ₙR₈ may be unsubstituted or substituted by one or two -(CH₂)ₙAr groups;
R4 is hydrogen, R₁₁, OH, C₁₋₅alkoxy, S(O)_{q}R₁₁, N(R₆)₂, -X(R₁₁), Br, F, I, Cl or NHCOR₆ wherein the C₁₋₅alkoxy may be unsubstituted or substituted by OH, methoxy or halogen;
R₆ is independently hydrogen or C₁₋₄alkyl;
R₇ is independently hydrogen, C₁₋₁₀alkyl, C₂₋₁₀alkenyl or C₂₋₈alkynyl all of which may be unsubstituted or substituted by one or more OH, N(R₆)₂, CO₂R₁₂, halogen or XC₁₋₅alkyl or R₇ is (CH₂)ₙAr;
R₈ is hydrogen, R₁₁, CO₂R₇, CO₂C(R₁₁)₂ O(CO)XR₇, PO₃(R₇)₂, SO₂NR₇R₁₁, NR₇SO₂R₁₁, CONR₇SO₂R₁₁, SO₃R₇, SO₂R₇, P(O)(OR₇)R₇, CN, -CO₂(CH₂)ₘC(O)N(R₆)₂, C(R₁₁)₂N(R₇)₂, C(O)N(R₆)₂, tetrazole or OR₆;
R₉ is (CH₂)ₙ, divalent C₁₋₁₀alkyl, divalent C₂₋₁₀alkenyl or phenyl, all of which may be unsubstituted or substituted by one or more OH, N(R₆)₂, COOH, halogen, or R₉ may be 〉C=O or XC₁₋₅alkyl;
R₁₀ is R₃ or R₄;
R₁₁ is hydrogen, Ar, C₁₋₈alkyl, C₂₋₈alkenyl, C₂₋₈alkynyl, all of which may be unsubstituted or substituted by one or more OH, CH₂OH, N(R₆)₂ or halogen; with the proviso that when R₁₁ is present in the group R₁₁CO₂R₇, R₁₁ is not hydrogen.
R₁₂ is hydrogen, C₁₋₆alkyl, C₂₋₆alkenyl or C₂₋₇alkynyl;
   X is (CH₂)ₙ, O, NR₆ or S(O)_{q};
Y is CH₃ or X(CH₂)ₙAr;
Ar is: 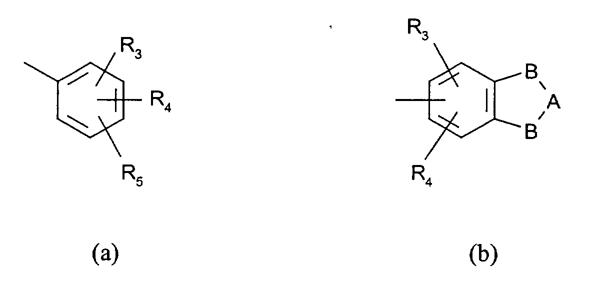
naphthyl, indolyl, pyridyl, thienyl, oxazolidinyl, oxazolyl, thiazolyl, isothiazolyl, pyrazolyl, triazolyl, tetrazolyl, imidazolyl, imidazolidinyl, thiazolidinyl, isoxazolyl, oxadiazolyl, thiadiazolyl, morpholinyl, piperidinyl, piperazinyl, pyrrolyl, or pyrimidyl; all of which may be unsubstituted or substituted by one or more R₃ or R₄ groups;
A is C=O, or [C(R₆)₂]ₘ;
B is -CH₂- or -O-;
Z₁ and Z₂ are independently hydrogen, C₁₋₈alkyl, C₂₋₈alkenyl, C₂₋₈alkynyl, OH, C₁₋₈alkoxy, S(O)_{q}C₁₋₈alkyl, N(R₆)₂, Br, F, I, Cl, NHCOR₆, -X-R₉-Y, -X(CH₂)ₙR₈, phenyl, benzyl or C₃₋₆cycloalkyl wherein the C₁₋₈alkyl, C₂₋₈alkenyl or C₂₋₈alkynyl may be optionally substituted by COOH, OH, CO(CH₂)ₙCH₃, CO(CH₂)ₙCH₂N(R₆)₂, or halogen; or Z₁ and Z₂ together may be -O-A-O- on contiguous carbons;
Z₃ is Z₁ or -X-R₉-Y;
q is zero, one or two;
n is an integer from 0 to six;
m is 1, 2 or 3; and the dotted line indicates the optional presence of a double bond; or a pharmaceutically acceptable salt thereof; provided that
   · R₂ is not hydrogen when X is S(O)_{q};
   · R₁ and R₇ are not hydrogen when q is 1 or 2 in S(O)_{q}R₁₁ or S(O)_{q}R₇;
   · when the optional double bond is present there is only one R₁₀ and there is no P₁ and P₂ is not NR₆R₉Y;
   · when the optional double bond is present in Formula (I) and X-R₂ is attached to the double bond, X is not NR₆;
   · when the optional double bond is present and R₁ is attached directly to the double bond, R₁ is not NR₆Ar;
   · when R₃, R₅, Z₁, Z₂, or Z₃ is X(CH₂)ₙR₈ and n is not 0, X is oxygen or NR₆ when R₈ is OR₆ or CO₂H;
   · when R₈ is CO₂C(R₁₁)₂O(CO)XR₇, X is not S(O)_{q};
   · the compound of Formula I is not methyl 1,3-diphenylindene-2-carboxylate; ethyl 1,3-diphenylindene-2-carboxylate; 1,3-diphenyl-2-cyanoindene; or ethyl 1,3-diphenyl-3-hydroxyindane-2-carboxylate;
   and further provided that compounds wherein:

R₁ is -X(CH₂)ₙAr or -X(CH₂)ₙR₈ or 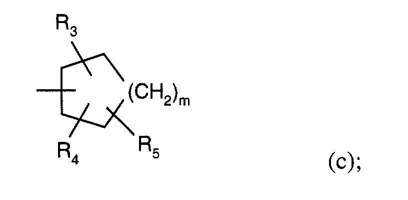
R₂ is hydrogen, Ar or (c);
P₁ is -X(CH₂)ₙR₈;
P₂ is -X(CH₂)ₙR₈, or -XR₉Y;
R₃ and R₅ are independently hydrogen, R₁₁, OH, C₁₋₈alkoxy, S(O)_{q}R₁₁, N(R₆)₂, Br, F, I, Cl, CF₃, NHCOR₆, -XR₉-Y or -X(CH₂)ₙR₈ wherein the methylene groups of -X(CH₂)ₙR₈ may be unsubstituted or substituted by one or more -(CH₂)ₙAr groups;
R₄ is hydrogen, R₁₁, OH, C₁₋₅alkoxy, S(O)_{q}R₁₁, N(R₆)₂, -X(R₁₁), Br, F, I, Cl or NHCOR₆ wherein the C₁₋₅alkoxy may be unsubstituted or substituted by OH, methoxy or halogen;
R₆ is independently hydrogen or C₁₋₄alkyl;
R₇ is independently hydrogen, C₁₋₆alkyl or (CH₂)ₙAr;
R₈ is hydrogen, R₁₁, CO₂H, PO₃H₂, P(O)(OH)R₇ or tetrazole;
R₉ is C₁₋₁₀alkyl, C₂₋₁₀alkenyl or phenyl all of which may be unsubstituted or substituted by one or more OH, N(R₆)₂, COOH, halogen or XC₁₋₅alkyl;
R₁₀ is R₃ or R₄;
R₁₁ is C₁₋₈alkyl, C₂₋₈alkenyl, C₂₋₈alkynyl all of which may be unsubstituted or substituted by one or more OH, CH₂OH, N(R₆)₂ or halogen;
X is (CH₂)ₙ, O, NR₆ or S(O)_{q};
Y is CH₃ or -CH₂X(CH₂)ₙAr;
Ar is: 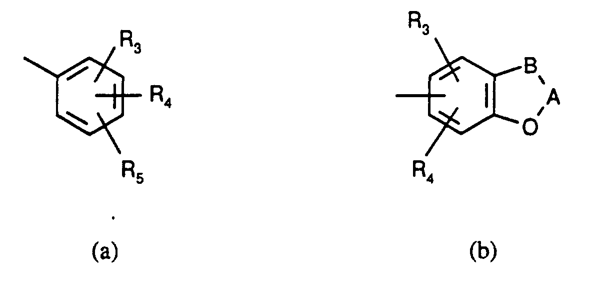
naphthyl, indolyl, pyridyl or thienyl, oxazolidinyl, oxazolyl, thiazolyl, isothiazolyl, pyrazolyl, triazolyl, tetrazolyl, imidazolyl, imidazolidinyl, thiazolidinyl, isoxazolyl, oxadiazolyl, thiadiazolyl, morpholinyl, piperidinyl, piperazinyl, pyrrolyl, or pyrimidyl; all of which may be unsubstituted or substituted by one or more R₃ or R₄ groups;
A is C=O, or [C(R₆)₂]ₘ;
B is -CH₂- or -O-;
Z₁ and Z₂ are independently hydrogen, C₁₋₈alkyl, C₂₋₈alkenyl, C₂₋₈alkynyl, OH, C₁₋₈alkoxy, S(O)_{q}C₁₋₈alkyl, N(R₆)₂, Br, F, I, Cl, NHCOR₆,
   -X(CH₂)ₙR₈, phenyl, benzyl or C₃₋₆cycloalkyl wherein the C₁₋₈alkyl, C₂₋₈alkenyl or C₂₋₈alkynyl may be optionally substituted by COOH, OH, CO(CH₂)ₙCH₃, CO(CH₂)ₙCH₂N(R₆)₂, or halogen; or Z₁ and Z₂ together may be -O-A-O- on contiguous carbons;
Z₃ is Z₁ or XR₉Y;
q is zero, one or two;
n is an integer from 0 to six;
m is 1, 2 or 3; are excluded.

Also included in the invention are pharmaceutically acceptable salt complexes.

All alkyl, alkenyl, alkynyl and alkoxy groups may be straight or branched. The term "halogen" is used to mean iodo, fluoro, chloro or bromo. Alkyl groups may be substituted by one or more halogens up to perhalogenation.

The compounds of the present invention may contain one or more asymmetric carbon atoms and may exist in racemic and optically active form. All of these compounds and diastereoisomers are contemplated to be within the scope of the present invention.

Preferred compounds are those wherein R₁ is X(CH₂)ₙAr, (Ar is (a) or (b)), dihydrobenzofuranyl, benzodioxanyl, cyclohexyl, C₁₋₄alkyl; R₂ is (a), (b) C₁₋₄alkyl, indolyl or hydrogen; R₃ and R₅ are independently hydrogen, OH, C₁₋₅alkoxy, halogen, -OC₁₋₄alkyl phenyl, R₁₁CO₂R₇, C₁₋₄alkyl, N(R₆)₂, NH(CO)CH₃, -X(CH₂)ₙR₈, -X-R₉-Y, pyridyl, phenyl or S(O)ₚC₁₋₅alkyl; R₄ is hydrogen, OH, C₁₋₅alkoxy, halogen, C₁₋₄alkyl, N(R₆)₂, NH(CO)CH₃ or S(O)ₚC₁₋₅alkyl; Z₁, Z₂ and Z₃ are independently XR₉Y, benzyl, hydrogen, OH, C₁₋₅alkoxy, -N(R₆)₂, S(O)_{q}C₁₋₈alkyl, NHCOR₆, X(CH₂)ₙR₈ or halogen, or Z₁ and Z₂ together may be -O-A-O- on contiguous carbons; P₁ and P₂ are independently hydrogen, CO₂H or tetrazole; Ar is (a), (b) or pyridyl; X is (CH₂)ₙ or oxygen.

More preferred are compounds wherein R₃ is hydrogen, -X(CH₂)ₙR₈ or R₁₁CO₂R₇; R₄ and R₅ are independently hydrogen, OH, C₁₋₅alkoxy, SC₁₋₅alkyl, a moiety of formula (a), F, Br, C₁₋₃alkyl or NH₂; Z₁ and Z₃ are hydrogen and Z₂ is hydrogen, OH, C₁₋₅alkoxy, halogen, X(CH₂)ₙR₈, NH₂, benzyl, NH(CO)CH₃, or Z₁ and Z₂ together may be O-A-O.

Most preferred are compounds wherein R₁ is (b) and R₂ is (a) or (b); A is CH₂, B is -O-; there is no optional double bond; R₁ and XR₂ are trans to P₁; Z₂ is OH, C₁₋₅alkoxy, -OCH₂CHCH₂ or hydrogen, Z₁ is hydrogen; R₃ is XAr, hydrogen, X(CH₂)_{q}COOH, X(CH₂)_{q}CONR₇SO₂R₁₁ or CH=CHCO₂H, R₄ is hydrogen, a moiety of formula (a) or C₁₋₂alkoxy; and R₅, R₁₀ and P₂ are hydrogen.

Also included are the following compounds:
(+) (1S,2R,3S)-3-(2-Carboxymethoxy-4-methoxyphenyl)-1-(3,4-methylenedioxyphenyl)-5-(prop-1-yloxy)indane-2-carboxylic acid;
(1RS, 2SR, 3RS)-3-[2-[(4-Carboxypyridin-3-yl)oxy]-4- methoxyphenyl]-1-(3,4-methylenedioxyphenyl)-5-(prop-1-yloxy)indane-2-carboxylic acid disodium salt;
(+) (1S, 2R, 3S)-3-[2-(2-Hydroxyeth-1-yloxy)-4-methoxyphenyl]-1-(3,4-methylenedioxyphenyl)-5-(prop-1-yloxy)indane-2-carboxylic acid, dicyclohexylamine salt;
(1S, 2R, 3S)-3-[(2,2-dimethylpropanoyloxymethoxycarbonylmethoxy)-4-methoxyphenyl)-1-(3,4-methylenedioxyphenyl)-5-(prop-1-yloxy)indane-2-carboxylic acid sodium salt.

The present invention provides compounds of Formula (I) above 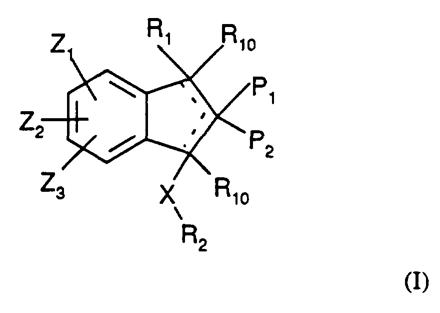 which can be prepared by a process which comprises:
a) reacting a compound of Formula (2) wherein X is C₁₋₅alkyl 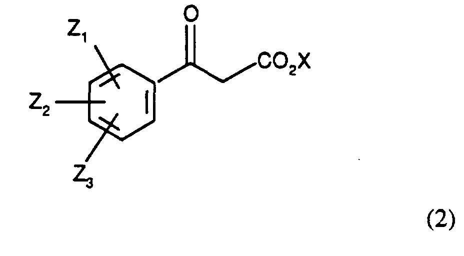 with a substituted benzaldehyde or aldehyde of Formula (3).

   D-CHO (3)

   wherein D is Ar or (c) as defined in Formula I, in a suitable solvent such as benzene with a catalyst such as piperidinium acetate at reflux to provide a compound of Formula (4). 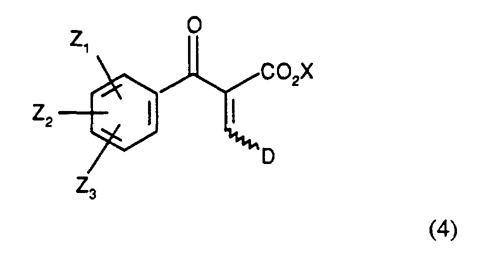
   Cyclization of compound (4) in the presence of a suitable Lewis acid such as titanium tetracholoride or aluminum chloride or alternatively when Z₁ is 3-OR (meta)(where R is C₁₋₅alkyl, or benzyl), trifluoroacetic acid, provides an indanone of the Formula (5). 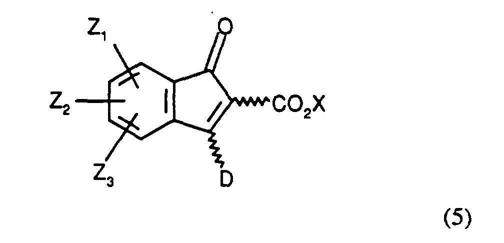
   Dehydrogenation with 2,3-dichloro-5,6-dicyano-1,4-benzoquinone in an appropriate solvent or alternatively bromination with pyridinium hydrobromide perbromide in dichloromethane followed by treatment with 1,5-diazabicyclo[4,3,0]non-5-ene provides indenones of Formula (6). 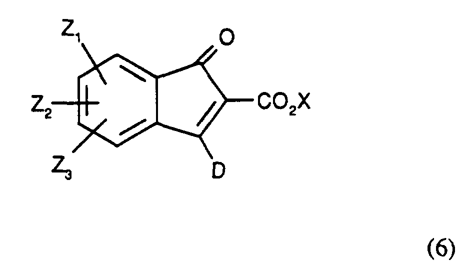
b) Alternatively, a compound of Formula 6 wherein Z₁, Z₂ and Z₃ are hydrogen and 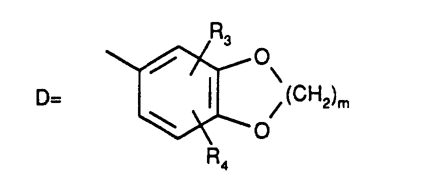 can be prepared by treatment of 2-bromobenzoic acid with two equivalents of n-butyllithium in a solvent such as tetrahydrofuran under argon at -78°C followed by the addition of an acid chloride of Formula (7): 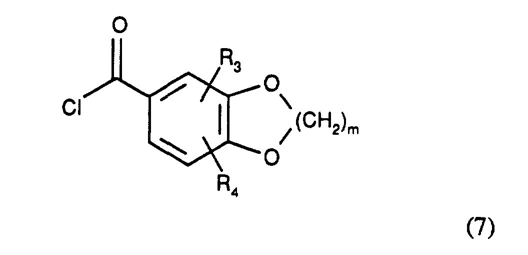 provides a compound of Formula (8): 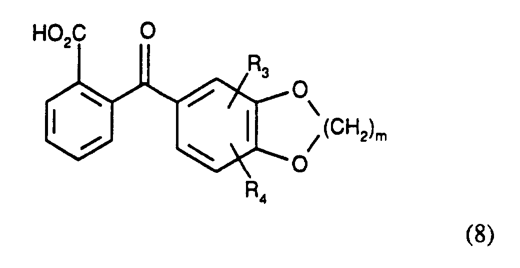
   Treatment of compounds of type (8) with thionyl chloride at reflux gives an acid chloride which can be isolated by concentration under reduced pressure. This acid chloride can then be treated with diethyl magnesium malonate in a solvent such as ether to give a compound of Formula (9): 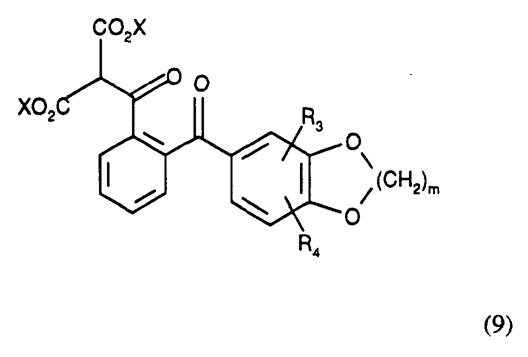
   Reaction of a compound of type (9) at reflux with 5% aqueous sodium carbonate gives compounds of Formula (10): 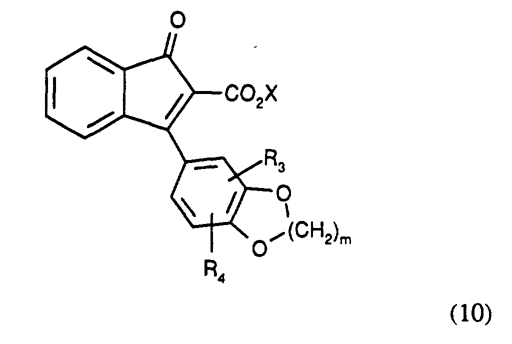
c) Treatment of an indenone of Formula (11): 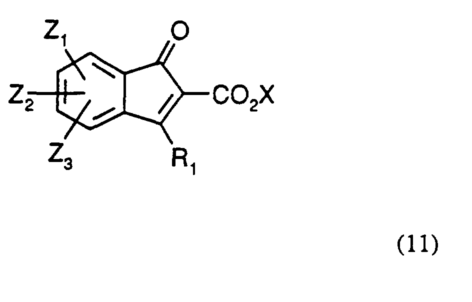 wherein Z₁, Z₂, Z₃ and R₁ are as defined for formula I or a group convertable to them, with an organomagnesium compound of Formula (12) wherein R₂ is defined for

   R₂(CH₂)ₙMgBr (12)

   Formula I or a group convertable to it, in a suitable solvent provides compounds of Formula (13):

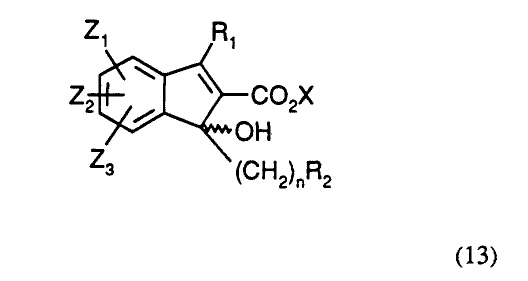

Saponification of compounds of Formula (13) using sodium hydroxide in aqueous methanol followed by reduction with triethylsilane and boron trifluoride etherate in a suitable solvent such as dichloromethane at 0°C affords racemic compounds of Formula (14). 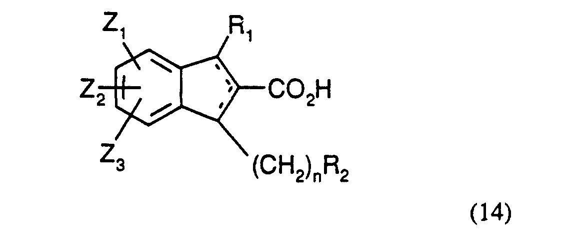 Conjugate addition of nucleophiles to an ester derived from Formula (14), followed by saponification affords compounds of Formula (I) having an R₁₀ other than hydrogen. Re-introduction of a double bond into an ester derived from such acids followed by conjugate addition of another nucleophilic species and subsequent saponification affords compounds of Formula (1) in which neither R₁₀ substituent is hydrogen.

Reduction of compounds of Formula (13) with triethylsilane and boron trifluoride etherate in a suitable solvent such as dichloromethane at 0°C followed by hydrogenation with hydrogen gas under pressure at approximately 60 psi in the presence of a suitable catalyst such as 10% palladium on charcoal affords compounds of Formula (15):

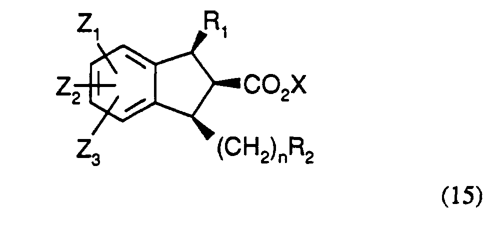

Alkylation or acylation of the ester enolate derived from Formula (15) affords compounds wherein P₁ and P₂ are as defined in Formula (1).

Alternatively, hydrogenation of compounds of Formula (13) with hydrogen gas under pressure at approximately 60 psi in the presence of a suitable catalyst such as 10% palladium on charcoal in a suitable solvent such as ethyl acetate or methanol containing 1-5% acetic acid affords compounds of Formula (15). Treatment of these compounds with a base such as sodium hydroxide in a suitable solvent such as aqueous ethanol provides racemic compounds of Formula (16): 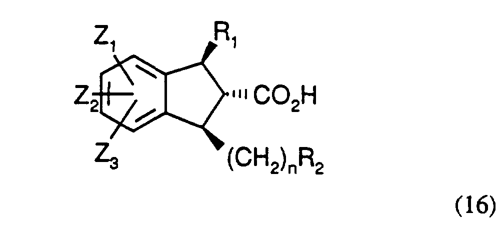 wherein Z₁, Z₂ and Z₃ are hydrogen; R₁ = R₂; and n is 0.
Treatment of compounds of Formula (13) with triethylsilane and boron trifluoride etherate in a suitable solvent such as dichloromethane at 0°C followed by reaction with samarium II iodide in a suitable solvent such as tetrahydrofuran and then saponification, provides compounds of Formula (17)

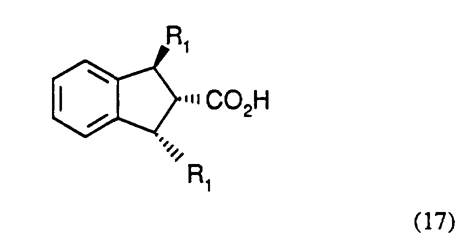

With appropriate manipulation and protection of any chemical functionalities, synthesis of the remaining compounds of the Formula (I) is accomplished by methods analogous to those above and to those described in the Experimental section.

In order to use a compound of the Formula (I) or a pharmaceutically acceptable salt thereof for the treatment of humans and other mammals it is normally formulated in accordance with standard pharmaceutical practice as a pharmaceutical composition.

Compounds of Formula (I) and their pharmaceutically acceptable salts may be administered in a standard manner for the treatment of the indicated diseases, for example orally, parenterally, sub-lingually, transdermally, rectally, via inhalation or via buccal administration.

Compounds of Formula (I) and their pharmaceutically acceptable salts which are active when given orally can be formulated as syrups, tablets, capsules and lozenges. A syrup formulation will generally consist of a suspension or solution of the compound or salt in a liquid carrier for example, ethanol, peanut oil, olive oil, glycerine or water with a flavouring or colouring agent. Where the composition is in the form of a tablet, any pharmaceutical carrier routinely used for preparing solid formulations may be used. Examples of such carriers include magnesium stearate, terra alba, talc, gelatin, agar, pectin, acacia, stearic acid, starch, lactose and sucrose. Where the composition is in the form of a capsule, any routine encapsulation is suitable, for example using the aforementioned carriers in a hard gelatin capsule shell. Where the composition is in the form of a soft gelatin shell capsule any pharmaceutical carrier routinely used for preparing dispersions or suspensions may be considered, for example aqueous gums, celluloses, silicates or oils and are incorporated in a soft gelatin capsule shell.

Typical parenteral compositions consist of a solution or suspension of the compound or salt in a sterile aqueous or non-aqueous carrier optionally containing a parenterally acceptable oil, for example polyethylene glycol, polyvinylpyrrolidone, lecithin, arachis oil, or sesame oil.

Typical compositions for inhalation are in the form of a solution, suspension or emulsion that may be administered as a dry powder or in the form of an aerosol using a conventional propellant such as dichlorodifluoromethane or trichlorofluoromethane.

A typical suppository formulation comprises a compound of Formula (1) or a pharmaceutically acceptable salt thereof which is active when administered in this way, with a binding and/or lubricating agent, for example polymeric glycols, gelatins, cocoa-butter or other low melting vegetable waxes or fats or their synthetic analogues.

Typical transdermal formulations comprise a conventional aqueous or non-aqueous vehicle, for example a cream, ointment, lotion or paste or are in the form of a medicated plaster, patch or membrane.

Preferably the composition is in unit dosage form, for example a tablet, capsule or metered aerosol dose, so that the patient may administer to themselves a single dose.

Each dosage unit for oral administration contains suitably from 0.1 mg to 500 mg/Kg, and preferably from 1 mg to 100 mg/Kg, and each dosage unit for parenteral administration contains suitably from 0.1 mg to 100 mg, of a compound of Formula (I) or a pharmaceutically acceptable salt thereof calculated as the free acid. Each dosage unit for intranasal administration contains suitably 1-400 mg and preferably 10 to 200 mg per person. A topical formulation contains suitably 0.01 to 1.0% of a compound of Formula (I).

The daily dosage regimen for oral administration is suitably about 0.01 mg/Kg to 40 mg/Kg, of a compound of Formula (I) or a pharmaceutically acceptable salt thereof calculated as the free acid. The daily dosage regimen for parenteral administration is suitably about 0.001 mg/Kg to 40 mg/Kg, of a compound of the Formula (I) or a pharmaceutically acceptable salt thereof calculated as the free acid. The daily dosage regimen for intranasal administration and oral inhalation is suitably about 10 to about 500 mg/person. The active ingredient may be administered from 1 to 6 times a day, sufficient to exhibit the desired activity.

No unacceptable toxicological effects are expected when compounds of the invention are administered in accordance with the present invention.

The biological activity of the compounds of Formula (I) are demonstrated by the following tests:

### I. Binding Assay

### A) Membrane Preparation

Rat cerebellum or kidney cortex were rapidly dissected and frozen immediately in liquid nitrogen or used fresh. The tissues, 1-2 g for cerebellum or 3-5 g for kidney cortex, were homogenized in 15 mls of buffer containing 20mM Tris HCl and 5mM EDTA, pH 7.5 at 4°C using a motor-driven homogenizer. The homogenates were filtered through cheesecloth and centrifuged at 20,000 x g for 10 minutes at 4°C. The supernatant was removed and centrifuged at 40,000 xg for 30 minutes at 4°C. The resulting pellet was resuspended in a small volume of buffer containing 50 mM Tris, 10 mM MgCl₂, pH 7.5; aliquotted with small vials and frozen in liquid nitrogen. The membranes were diluted to give 1 and 5 mg of protein for each tube for cerebellum and kidney cortex in the binding assay.

Freshly isolated rat mesenteric artery and collateral vascular bed were washed in ice cold saline (on ice) and lymph nodes were removed from along the major vessel. Then, the tissue was homogenized using a polytron in buffer containing 20 mM Tris and 5mM EDTA, pH 7.5 at 4°C in 15 ml volume for ∼6 gm of mesenteric artery bed. The homogenate was strained through cheesecloth and centrifuged at 2,000 xg for 10 min. at 4°C. The supernatant was removed and centrifuged at 40,000 xg for 30 min. at 4°C. The resulting pellet was resuspended as explained above for cerebellum and kidney cortex. Approximately 10 mg of membrane protein was used for each tube in binding experiments.

### B) [¹²⁵I]ET-1 Binding Protocol

[¹²⁵I]ET-1 binding to membranes from rat cerebellum (2-5 mg protein/assay tube) or kidney cortex (3-8 mg protein/assay tube) were measured after 60 minutes incubation at 30°C in 50 mM Tris HCl, 10 mM MgCl₂, 0.05% BSA, pH 7.5 buffer in a total volume of 100 ml. Membrane protein was added to tubes containing either buffer or indicated concentration of compounds. [¹²⁵I]ET-1 (2200 Ci/mmol) was diluted in the same buffer containing BSA to give a final concentration of 0.2-0.5 nM ET-1. Total and nonspecific binding were measured in the absence and presence of 100 nM unlabelled ET-1. After the incubation, the reactions were stopped with 3.0 ml cold buffer containing 50 mM Tris and 10 mM MgCl₂, pH 7.5. Membrane bound radioactivity was separated from free ligand by filtering through Whatman GF/C filter paper and washing the filters 5 times with 3 ml of cold buffer using a Brandel cell harvester. Filter papers were counted in a gamma counter with an efficiency of 75%. IC₅₀'s for the compounds of this invention range from 0.1 nm to 50 mm.

### II. In Vitro Vascular Smooth Muscle Activity

Rat aorta are cleaned of connective tissue and adherent fat, and cut into ring segments approximately 3 to 4 mm in length. Vascular rings are suspended in organ bath chambers (10 ml) containing Krebs-bicarbonate solution of the following composition (millimolar): NaCl, 112.0; KCl, 4.7; KH₂PO₄, 1.2; MgSO₄, 1.2; CaCl₂, 2.5; NaHCO₃, 25.0; and dextrose, 11.0. Tissue bath solutions are maintained at 37°C and aerated continuously with 95% 0₂/ 5% CO₂. Resting tensions of aorta are maintained at 1 g and allowed to equilibrate for 2 hrs., during which time the bathing solution is changed every 15 to 20 min. Isometric tensions are recorded on Beckman R-611 dynographs with Grass FT03 force-displacement transducer. Cumulative concentration-response curves to ET-1 or other contractile agonists are constructed by the method of step-wise addition of the agonist. ET-1 concentrations are increased only after the previous concentration produces a steady-state contractile response. Only one concentration-response curve to ET-1 is generated in each tissue. ET receptor antagonists are added to paired tissues 30 min prior to the initiation of the concentration-response to contractile agonists.

ET-1 induced vascular contractions are expressed as a percentage of the response elicited by 60 mM KCl for each individual tissue which is determined at the beginning of each experiment. Data are expressed as the mean ± S.E.M. Dissociation constants (K_{b}) of competitive antagonists were determined by the standard method of Arunlakshana and Schild. The potency range for compounds of this invention range from 0.1 nM to 50 mm.

The following examples are illustrative and are not limiting of the compounds of this invention.

### EXAMPLE 1

### (+) (1S,2R,3S)-3-(2-Carboxymethoxy-4-methoxyphenyl)-1-(3,4-methylenedioxyphenyl)-5-(prop-1-yloxy)indane-2-carboxylic acid

a) 3-(Prop-1-yloxy)acetophenone. To a slurry of NaH (13.84 g, 0.58 mol) in dry DMF (50 mL) at 0°C, was added a solution of 3-hydroxyacetophenone (50 g, 0.37 mol). After stirring for 30 min. 1-iodopropane (70 mL, 0.72 mol) was added and the mixture stirred overnight at room temperature. The mixture was diluted with dry DMF (50 mL) and further NaH (2.77 g, 0.12 mol) added followed by 1-iodopropane (23 mL, 0.24 mol). After 1 h. TLC indicated that the reaction was complete and the product was cautiously quenched with 6M HCl and extracted with EtOAc. The EtOAc extract was washed successively with H₂O, 10% aqueous NaOH and then brine. After drying (MgSO₄), filtration and evaporation gave the title compound (65 g, 98%) as a light yellow oil which was used without further purification. Anal. Calc. for C₁₁ H₁₄O₂: C, 74.13; H, 7.89. Found: C, 73.85; H, 7.86.
b) Methyl 3-(Prop-1-yloxy)benzoylacetate. To a suspension of NaH (12 g, 0.5 mol) in dry dimethyl carbonate (50 mL) was added slowly a solution of 3-(Prop-1-yloxy)acetophenone (65 g, 0.37 mol) in dry dimethyl carbonate (100 mL). During the addition the exothermicity of the reaction caused refluxing. Following the addition the mixture was stirred mechanically overnight and was then quenched cautiously with 3M HCl and extracted with EtOAc. The EtOAc extract was washed successively with H₂O, 5% aqueous NaHCO₃, H₂O and brine. After drying (MgSO₄), filtration and evaporation gave a yellow oil (82 g, quantitative) which was used without further purification.
c) Methyl 3-(3,4-methylenedioxyphenyl)-2-[3-(prop-1-yloxy)-benzoyl]propenoate.To a solution of methyl 3-(prop-1-yloxy)benzoylacetate (10 g, 4.2 mmol) in benzene (50 mL) was added 3,4-methylenedioxybenzaldehyde (6.36 g, 4.2 mmol) followed by piperidine (0.42 mL, 0.42 mmol) and glacial acetic acid (8 drops approx.). The mixture was refluxed for 2 h. and the volatiles removed *in vacuo* to give methyl 3-(3,4-methylenedioxyphenyl)-2-[3-(prop-1-yloxy)-benzoyl]propenoate (7.4 g, 48%) as an off white solid after trituration with methanol (m. p. 122-123°C). Anal. Calc. for C₂₁ H₂₀O₆: C, 68.47; H, 5.47. Found: C, 68.81; H, 5.49.
d) Methyl (1RS,2SR)-1-(3,4-Methylenedioxyphenyl)-5-(prop-1-yloxy)-3-oxo-indane-2-carboxylate. Methyl 3-(3,4-methylenedioxyphenyl)-2-[3-(prop-1-yloxy)-benzoyl]propenoate (7.4 g, 2.0 mmol) was dissolved in trifluoroacetic acid (50 mL) at 0°C and the mixture stirred at room temperature for 20 min. The trifluoroacetic acid was removed *in vacuo* to give the title compound (6.4 g, 87 %) as a white solid after trituration with warm isopropanol m. p. 106-108°C. Anal. Calc. for C₂₁ H₂₀O₆: C, 68.47; H, 5.47. Found: C, 68.12; H, 5.41.
e) Methyl 3-(3,4-Methylenedioxyphenyl)-6-(prop-1-yloxy)-1-oxo-indene-2-carboxylate. Methyl (1RS, 2SR)-1-(3,4-methylenedioxyphenyl)-5-(prop-1-yloxy)-3-oxo-indane-2-carboxylate (26.2 g, 71 mmol) was dissolved in toluene (250 mL) and DDQ (dichlorodicyano-quinone) (16.5 g, 71 mmol) was added. The mixture was heated at 80°C for 2 h. then cooled, filtered and the solvent removed *in vacuo.* The product was purified by flash column chromatography on silica gel (eluant: EtOAc/hexane, 20:80) to give the title compound as an orange solid (11.3 g, 44 %); m.p. 125-126°C. Anal. Calc. for C₂₁ H₁₈O₆: C, 68.85; H, 4.95. Found: C, 68.45; H, 4.97.
f) Methyl (1RS)-1-Hydroxy-1-(4-methoxy-2-methoxymethoxyphenyl)-3-(3,4-methylenedioxyphenyl)-6-(prop-1-yloxy)indene-2-carboxylate. To dry magnesium turnings (1.7 g, 69 mmol) under an argon atmosphere was added portionwise, a solution of 1-bromo-4-methoxy-2-methoxymethoxybenzene (16.8 g, 68 mmol) in 5% THF/ether (120 mL). The resulting 4-methoxy-2-methoxymethoxyphenyl magnesium bromide was added to a solution of methyl 3-(3,4-methylenedioxyphenyl)-6-(prop-1-yloxy)-1-oxo-indene-2-carboxylate (18.5 g, 51 mmol) in THF (400 mL) under an argon atmosphere at 0°C. The resulting mixture was allowed to warm to room temperature and was stirred for 10 min. The mixture was partitioned between 3M HCl and EtOAc . The organic extract was washed successively with H₂O, aqueous NaHCO₃, H₂O and saturated aqueous NaCl and dried (Na₂SO₄). The solvent was removed under reduced pressure, and the residue purified by flash chromatography on silica gel (eluant: EtOAc/hexane, 10-20%) to afford the title compound as a yellow oil (24.5 g, 91%). Anal. Calc. for C₃₀H₃₀O₉: C, 67.41; H, 5.66;. Found: C, 67.21; H, 5.66.

### SEPARATION

Separation of (+) and (-) methyl (1RS)-1-Hydroxy-1-(4-methoxy-2-methoxymethoxyphenyl)-3-(3,4-methylenedioxyphenyl)-6-(prop- 1-yloxy)indene-2-carboxylate was done on a column of cellulose tris(3,5-dimethylphenyl carbamate) coated on silica gel (Daicel Chiralcel OD); retention time for (+) 8.8 min. [α]²⁵_{D}= +87.5° (c = 0.24, CH₃OH). Retention time for (-) 14.5 min. [α]²⁵_{D}= +85.9° (c = 0.21, CH₃OH)
HPLC data: column Chiralcel OD (DAICEL) 21.2 mm internal diameter, 250 mm length; solvent Ethanol:Hexane 60:40; flow rate 10 mL/min.; injection: 1 g of racemate; detection UV = 405 nm
g) (+)Methyl (1S,2S,3S)-3-(4-Methoxy-2-methoxymethoxyphenyl)-1-(3,4-methylenedioxyphenyl)-5-(prop-1-yloxy)indane-2-carboxylate. A parr vessel was charged with (+) methyl (1RS)-1-Hydroxy-1-(4-methoxy-2-methoxymethoxyphenyl)-3-(3,4-methylenedioxyphenyl)-6-(prop-1-yloxy)indene-2-carboxylate (1 g, 1.8 mmol) dissolved in a small volume of EtOAc (25 mL) and 10% palladium on activated carbon (93 mg). The resulting solution was stirred under an atmosphere of hydrogen for 120 hours and filtered. The filtrate was concentrated under reduced pressure and the product purified by column chromatography on silica gel (eluant: EtOAc/hexane, 5-10%) to give the title compound as a white foam (0.80 g, 83%). [α]²⁵_{D}= +105.4° (c = 0.13, CH₃OH). Anal. Calc. for C₃₀H₃₂O₈: C, 69.22; H, 6.20. Found: C, 68.95; H, 6.11.
h) (+)methyl (1S,2S,3S)-3-(2-Hydroxy-4-methoxyphenyl)-1-(3,4-methylenedioxyphenyl)-5-(prop-1-yloxy)indane-2-carboxylate. To a solution of methyl (1S,2S,3S)-3-(4-Methoxy-2-methoxymethoxyphenyl)-1-(3,4-methylenedioxyphenyl)-5-(prop-1-yloxy)indane-2-carboxylate (0.7 g, 1.3 mmol) in methanol (10 mL) concentrated HCl (0.1 mL) was added and it was then heated to reflux for 2 h. The solvent was then eliminated under vacuum and the residue was purified by column chromatography on silica gel (eluant: EtOAc/hexane, 10-20%) to give the title compound as a colorless glass (0.50 g, 78%). [α]²⁵_{D}= +116.0° (c = 0.18, CH₃OH). Anal. Calc. for C₂₈H₂₈O₇.1/2 H₂O: C, 69.27; H, 6.02.
   Found: C, 69.59; H, 5.99.
i) (+) Methyl (1S,2S,3S)-3-(2-Carboethoxymethoxy-4-methoxyphenyl)-1-(3,4-methylenedioxyphenyl)-5-(prop-1-yloxy)indane-2-carboxylate. A solution of methyl (1S,2S,3S)-3-(2-hydroxy-4-methoxyphenyl)-1-(3,4-methylenedioxyphenyl)-5-(prop-1-yloxy)indane-2-carboxylate (0.1 g, 0.2 mmol) in dry DMF (2 mL) was added to NaH (6 mg, 0.24 mmol) in a small volume of dry DMF at 0°C. The mixture was stirred at 0°C for 15 min. and ethyl bromoacetate was then added (42 mg, 0.25 mmol). The resulting mixture was stirred at 0°C for 1h. The reaction was then quenched with dilute HCl and extracted with EtOAc. The EtOAc extract was washed with water then brine, dried (MgSO₄), filtered and evaporated. The product was purified by column chromatography on silica gel (eluant: EtOAc/hexane, 10-15%) to give the title compound as a glassy solid (82 mg, 68%).[α]²⁵_{D}= +116.0° (c = 0.45, CH₃OH). Anal. Calc. for C₃₂ H₃₄O₉: C, 68.32; H, 6.09. Found: C, 67.98; H, 6.09.
j) (+) (1S,2R,3S)-3-(2-Carboxymethoxy-4-methoxyphenyl)-1-(3,4-methylenedioxyphenyl)-5-(prop-1-yloxy)indane-2-carboxylic acid. To a solution of methyl (1S,2S,3S)-3-(2-carboethoxymethoxy-4-methoxyphenyl)-1-(3,4-methylenedioxyphenyl)-5-(prop-1-yloxy)indane-2-carboxylate (20 mg, 0.04 mmol) in dioxane (1 mL) was added 3 M NaOH solution (0.3 mL, 1 mmol). The reaction mixture was heated to reflux for 4 h and after cooling the solvent was eliminated in vacuo dissolved in water and acidified with 3N HCl. The resulting precipitate was collected by filtration and dried to give a white solid (15 mg, 81%); m.p. 99-102°C [α]²⁵D= +38.1° (c = 0.22, CH₃OH). Anal. Calc. for C₂₉H₂₈ O₉: C, 66.92; H, 5.42. Found C, 67.37; H, 5.32.

### EXAMPLE 1A

### Preparation of 1-Bromo-4-methoxy-2-methoxymethoxybenzene.

a) 1-Bromo-2-hydroxy-4-methoxybenzene. 3-Bromo-2-hydroxy-6-methoxybenzoic acid [T. de Paulis et. al. ,J. Med. Chem., (1985), 28, 1263-1269] (5 g, 0.02 mol) was heated in quinoline (200 mL) at 160°C for 1 h. On cooling, the product was partitioned between Et₂O and 3M HCl. The organic extract was washed with water and brine then dried (MgSO₄), filtered and evaporated to give the title compound which was recrystallized from 5% ethyl acetate/hexane (4 g, 97%); m.p. 40-42°C. Anal. Calc. for C₇H₇ BrO₂: C, 41.41; H, 3.48.
   Found C, 41.39; H, 3.37.
b) 1-Bromo-4-methoxy-2-methoxymethoxybenzene. To a suspension of NaH (2.5 g, 0.06 mol) in dry DMF (100 mL) at 0°C was added solution of 1-bromo-2-hydroxy-4-methoxybenzene (10.6 g, 0.05 mol). After stirring at 0°C for 30 min. bromomethyl methyl ether (7.8 g, 0.06 mmol) were dropwise added. The mixture was warmed to room temperature over 20 min. and then stirred for 2 h, it was then quenched cautiously by the addition of cold dilute HCl and extracted with EtOAc. The EtOAc extract was washed successively with; H₂O, 5% aqueous NaHCO₃, H₂O and finally brine. After drying (MgSO₄) filtration and evaporation gave liquid. The product was purified by distillation (85°C, 0.2 mm Hg) to give the title compound as a colorless oil (13.7 g, 97%).
   ¹H NMR (CDCl₃) δ 7.40 (d, 1 H, J = 8.9 Hz), 6.75 (d, 1 H, J = 2.8 Hz), 6.46 (dd, 1 H, J = 8.9, 2.8 Hz), 5.23 (s, 2 H), 3.77 (s, 3 H), 3.52 (s, 3 H).

### EXAMPLE 2

### (1RS,2SR,3RS)-3-[2-(2-Hydroxyeth-1-yloxy)-4-methoxyphenyl]-1-(3,4-methylenedioxyphenyl)-5-(prop-1-yloxy)indane-2-carboxylic acid. dicyclohexylamine salt

m.p. 182-184°C.
Anal. Calc. for C₄₁H₅₃NO₈: C, 71.59; H, 7.77;
N, 2.04. Found: C, 71.67; H, 7.66; N, 2.42.

### EXAMPLE 2A

### (+) (1S,2R,3S)-3-[2-(2-Hydroxyeth-1-yloxy)-4-methoxyphenyl]-1-(3,4-methylenedioxyphenyl)-5-(prop-1-yloxy)indane-2-carboxylic acid

a) (+) Methyl-(1S,2R,3S)-3-[2-(2-*t*-Butyldimethylsiloxyeth-1-yloxy)-4-methoxyphenyl]-1-(3,4-methylenedioxyphenyl)-5-(prop-1-yloxy)indane-2-carboxylate. A solution of methyl-(1S,2S,3S)-3-(2-hydroxy-4-methoxyphenyl)-1-(3,4-methylenedioxyphenyl)-5-(prop-1-yloxy)indane-2-carboxylate (3 g, 6 mmol) in dry DMF (30 mL) was added to NaH (230 mg, 7 mmol, 80%) in a small volume of dry DMF at 0°C. The mixture was stirred at 0°C for 15 min. and 2-*t*-butyldimethylsiloxyethylbromide was then added dropwise (1.65 g, 8 mmol). The resulting mixture was stirred at 0°C for 3 h. The reaction was then quenched with dilute HCl and extracted with EtOAc. The EtOAc extract was washed with water then brine, dried (MgSO₄), filtered and evaporated. The product was purified by column chromatography on silica gel (eluant: EtOAc/hexane, 5-10%) to give the title compound as a colorless oil (520 mg, 18% based on recovery of epimerized starting material).
b) Methyl-(+) (1S,2R,3S)-3-[2-(2-Hydroxyeth-1-yloxy)-4-methoxyphenyl]-1-(3,4-methylenedioxyphenyl)-5-(prop-1-yloxy)indane-2-carboxylate. To a solution of (+) methyl-(1S, 2R, 3S)-3-[2-(2-t-butyldimethylsiloxyeth-1-yloxy)-4-methoxyphenyl]-1-(3,4-methylenedioxyphenyl)-5-(prop-1-yloxy)indane-2-carboxylate (0.520 g, 0.74 mmol) in THF (10 mL) at RT, a 1 M solution of tetra-*n*-butylammonium fluoride (2.5 mL) in THF was added. The reaction mixture was stirred at RT for 1 h. After concentration in vacuo, the residue was partitioned between ether/ethyl acetate and water. The organic layer was washed with brine, dried (MgSO₄) and concentrated. The residue was purified by flash column chromatography on silica gel (eluant: 20-30% ethyl acetate/hexane) to give the title compound as a colorless foam (0.35 g, 82%).
c) (+) (1S, 2R, 3S)-3-[2-(2-Hydroxyeth-1-yloxy)-4-methoxyphenyl]-1-(3,4-methylenedioxyphenyl)-5-(prop-1-yloxy)indane-2-carboxylic acid To a solution of methyl-(1S,2R,3S)-3-[2-(2-hydroxyeth-1-yloxy)-4-methoxyphenyl)-1-(3,4-methylenedioxyphenyl)-5-(prop-1-yloxy)indane-2-carboxylate (0.35 g, 0.67 mmol) in methanol/THF 1/2 (15 mL) was added a 0.5 M LiOH solution (5 mL). The reaction mixture was stirred at RT overnight. The solvent was eliminated in vacuo the product dissolved in water and acidified with 3N HCl. The resulting precipitate was collected by filtration and dried to give a white solid (0.31 mg, 91%); m.p. 94-98°C.[α]²³_{D}= +73.16° (c = 0.19, CH₃OH) Anal. Calc. for C₂₉H₂₉O₈Na.11/8 H₂O: C, 63.47; H, 5.74. Found: C, 63.39; H, 5.66.

### EXAMPLE 3

### (1RS,2SR,3RS)-3-[2-[(4-Carboxypyridin-3-yl)oxy]-4-methoxyphenyl]-1-(3,4-methylenedioxyphenyl)-5-(prop-1-yloxy)indane-2-carboxylic acid disodium salt:

### a) Methyl(1RS,2SR,3RS)-3-[2-[(4-Formylpyridin-3-yl)oxyl-4-methoxyphenyl]-1-(3,4-methylenedioxyphenyl)-5-(prop-1-yloxy)indane-2-carboxylate

To a solution of Methyl (1RS, 2SR, 3RS)-3-(2-hydroxy-4-methoxyphenyl)-1-(3,4-methylenedioxyphenyl)-5-(prop-1-yloxy)indane-2-carboxylate (300 mg, 0.63 mmol) in DMF (4 mL) was added K₂CO₃ (109 mg, 0.79 mmol) and 3-fluoro-4-formylpyridine (150 mg, 1.2 mmol). The reaction mixture was heated to reflux under argon for 2 h. After cooling to room temperature it was partitioned between 3N HCl and ethyl acetate. The ethyl acetate extract was washed with water, aqueous NaHCO₃ and brine and dried (MgSO₄). The solvent was removed *in vacuo* and the residue purified by flash column chromatography (silica gel, gradient elution from 10% to 20% ethyl acetate/hexanes) to afford the title compound (128 mg, 42%).

### b) Methyl (1RS, 2SR, 3RS)-3-[2-[(4-Carboxypyridin-3-yl)oxy]-4-methoxyphenyl]-1-(3,4-methylenedioxyphenyl)-5-(prop-1-yloxy)indane-2-carboxylate

To a solution of Methyl (1RS, 2SR, 3RS)-3-[2-[(4-formylpyridin-3-yl)oxy]-4-methoxyphenyl]-1-(3,4-methylenedioxyphenyl)-5-(prop-1-yloxy)indane-2-carboxylate (128 mg, 0.24 mmol) in t-BuOH (10 mL) was added a solution of NaClO₂ (34 mg, 0.28 mmol) and NH₂SO₃H (40 mg, 0.42 mmol) in water (6mL). The reaction mixture was stirred at room temperature for 2 h and partitioned between water and ethyl acetate. The ethyl acetate extract was washed with water and brine and dried (MgSO₄). The solvent was removed *in vacuo* the residue was purified by flash column chromatography (silica gel, 25% ethyl acetate/hexanes containing 5% of acetic acid) to afford the title compound (90 mg, 69%).

### c) (1RS,2SR,3RS)-3-[2-[(4-Carboxypyridin-3-yl)oxy]-4-methoxyphenyl]-1-(3,4-methylenedioxyphenyl)-5-(prop-1-yloxy)indane-2-carboxylic acid disodium salt

To a solution of Methyl (1RS, 2SR, 3RS)-3-[2-[(4-Carboxypyridin-3-yl)oxy]-4-methoxyphenyl]-1-(3,4-methylenedioxyphenyl)-5-(prop-1-yloxy)indane-2-carboxylate (90, 0.15 mmol) in isopropanol (2 mL) was added 1M aqueous NaOH (0.3 mL, 0.3 mmol). The resulting mixture was heated to reflux for 12 h, then concentrated under reduced pressure. The residue was partitioned between dilute HCl and ethyl acetate. The ethyl acetate extract was washed with water and dried (MgSO₄). The solvent was removed *in vacuo* and the residue was purified by flash column chromatography (silica gel, 30% ethyl acetate/hexanes containing 5% of acetic acid) to afford the title compound (65 mg, 74%); m.p. 220-222°C (dec.) (disodium salt).

### EXAMPLE 4

### 2,2-Dimethylpropanoyloxymethyl (1RS,2SR,3RS)-3-(2-carboxymethoxy-4-methoxyphenyl)-1-(3,4-methylenedioxyphenyl)-5-(prop-1-yloxy)indane-2-carboxylate sodium salt

### a) 2,2-Dimethylpropanoyloxymethyl (1RS,2SR,3RS)-3-(2-hydroxy-4-methoxyphenyl)-1-(3,4-methylenedioxyphenyl)-5-(prop-1-yloxy)indane-2-carboxylate

(1RS,2SR,3RS)-3-(2-hydroxy-4-methoxyphenyl)-1-(3,4-methylenedioxyphenyl)-5-(prop-1-yloxy)indane-2-carboxylic acid potassium salt (125 mg, 0.54 mmol) (obtained by treatment of the corresponding acid with KHCO₃ (54 mg, 0.54 mmol)) was dissolved in DMF (3 ml) and pivaloyloxymethyl iodide (0.54 mmol) (prepared from pivaloyloxymethyl chloride (73 mg, 0.54 mmol) and excess sodium iodide in acetone) added. The reaction mixture was stirred overnight and then partitioned between dil. HCl and ethyl acetate. The organic layer was washed with water and brine then dried (MgSO₄ anhyd.) filtered and evaporated. The product was purified by column chromatography to provide the title compound (120 mg, 77%) as a colorless oil.

### b) 2,2-Dimethylpropanoyloxymethyl (1RS,2SR,3RS)-3-(2-carbobenzyloxymethoxy-4-methoxyphenyl)-1-(3,4-methylenedioxyphenyl)-5-(prop-1-yloxy)indane-2-carboxylate.

2,2-Dimethylpropanoyloxymethyl (1RS,2SR,3RS)-3-(2-hydroxy-4-methoxyphenyl)-1-(3,4-methylenedioxyphenyl)-5-(prop-1-yloxy)indane-2-carboxylate (280 mg, 0.5 mmol) in dry DMF (3 ml) was added to NaH (18 mg, 0.6 mmol) in a small volume of dry DMF. The mixture was stirred at RT for 20 min. then benzyl bromoacetate (137 mg, 0.6 mmol) was added. After stirring for 1.5 h the product was partitioned between 3M aqueous HCl and ethyl acetate. The organic layer was washed with water then brine, dried (MgSO₄ anhyd.) filtered and evaporated to give an oil. The product was purified by column chromatography to provide the title compound (240 mg, 66%) as a colorless oil.

### c) 2.2-Dimethylpropanoyloxymethyl (1RS,2SR,3RS)-3-(2-carboxymethoxy-4-methoxyphenyl)-1-(3,4-methylenedioxyphenyl)-5-(prop-1-yloxy)indane-2-carboxylic acid.

2,2-Dimethylpropanoyloxymethyl (1RS,2SR,3RS)-3-(2-carbobenzyloxymethoxy-4-methoxyphenyl)-1-(3,4-methylenedioxyphenyl)-5-(prop-1-yloxy)indane-2-carboxylate (240 mg, 0.3 mmol) was dissolved in a 2:1 mixture of ethyl acetate and ethanol (3 ml) and then 50 mg of 10%Pd/C was added. The mixture was stirred at room temperature under an H₂ atmosphere for 3 h. The catalyst was then filtered, the solvent concentrated *in vacuo* and the resultant oil purified by flash column chromatography. The title compound was obtained (180 mg, 86%) as a colorless oil.
MS (exact mass) (M+Na)⁺·: 647.2335 (sodium salt)
(D = -2.3. mDa for C₃₃H₃₈O₁₁Na)
mp 190-195°C (dec, sodium salt)

### EXAMPLE 5

### (1S,2R,3S)-3-[2-[Carbo-(1RS)-1-(2-methoxy-2-methylpropionyloxy)eth-1-yloxymethoxy]-4-methoxyphenyl-1-(3,4-methlenedioxyphenyl)-5-prop-1-yloxy)indane-2-carboxylic acid sodium salt

### a) Allyl (1S,2R,3S)-3-(4-methoxy-2-methoxymethoxyphenyl)-1-(3,4-methylenedioxyphenyl)-5-(prop-1-yloxy)indane-2-carboxylate.

(1S, 2R, 3S)-3-(4-methoxy-2-methoxymethoxyphenyl)-1-(3,4-methylenedioxyphenyl)-5-(prop-1-yloxy)indane-2-carboxylic acid (4.8 g, 9.5 mmol) was dissolved in dry acetonitrile (30 ml) and DBU (1.7 ml, 11.4 mmol) was added followed by allyl bromide (3.4 g, 28. mmol). After stirring for 0.5 h. the product was partitioned between 3M aqueous HCl and ethyl acetate. The organic layer was washed with water and brine, then dried (MgSO₄ anhyd.) filtered and evaporated to give an oil. The product was purified by column chromatography to provide the title compound as a pale yellow oil (5.7 g, quantitative).

### b) Allyl (1S, 2R, 3S)-3-(2-hydroxy-4-methoxyphenyl)-1-(3,4-methylenedioxyphenyl)-5-(prop-1-yloxy)indane-2-carboxylate.

Allyl (1S, 2R, 3S)-3-(4-methoxy-2-methoxymethoxyphenyl)-1-(3,4-methylenedioxyphenyl)-5-(prop- -yloxy)indane-2-carboxylate (1.7 g, 3.11 mmol) was dissolved in allyl alcohol (20 ml) and then 15 drops of conc. HCl was added. The resulting solution was stirred at 65°C for 2 h. After removing the solvent the residue was partitioned between water and ethyl acetate. The organic layers were washed with water, 5% aqueous NaHCO₃ and brine; then dried (MgSO₄ anhyd.), filtered and evaporated to give an oil. The product was purified by column chromatography to provide the title compound as a pale yellow oil (1.26 g, 81%).

### c) Allyl (1S, 2R, 3S)-3-(2-Carbo-1-tert-butoxymethoxy-4-methoxyphenyl)-1-(3,4-methylenedioxyphenyl)-5-(prop-1-yloxy)indane-2-carboxylate.

Allyl (1S, 2R, 3S)-3-(2-Hydroxy-4-methoxyphenyl)-1-(3,4-methylenedioxyphenyl)-5-(prop-1-yloxy)indane-2-carboxylate (1.0 g, 2 mmol) in dry DMF (4 ml) was added to NaH (57 mg, 2.4 mmol) in a small volume of dry DMF. The mixture was stirred at RT for 20 min., then *tert*-butyl bromoacetate (974 mg, 5 mmol) was added. After stirring for 0.5 h., the product was partitioned between 3M aqueous HCl and ethyl acetate. The organic layer was washed with water, brine and dried (MgSO₄ anhyd.), filtered and evaporated to give an oil. The product was purified by column chromatography to provide the title compound (1.1 g, 93%) as a pale yellow oil.

### d) Allyl (1S, 2R, 3S)-3-(2-carboxymethoxy-4-methoxyphenyl)-1-(3,4-methylenedioxyphenyl)-5-(prop-1-yloxy)indane-2-carboxylate.

Allyl (1S, 2R, 3S)-3-(2-carbo-tert-butoxymethoxy-4-methoxyphenyl)-1-(3,4-methylenedioxyphenyl)-5-(prop-1-yloxy)indane-2-carboxylate (765 mg, 1.24 mmol) was dissolved in TFA (5 ml) containing a few drops of anisole. The reaction mixture was stirred at RT for 20 min. The solvent was eliminated, the residue was diluted with ethyl acetate, washed with water, brine and dried (MgSO₄ anhyd.), filtered and evaporated. The product was purified by column chromatography to provide the title compound (575 mg, 83%) as a colorless oil.

### e) Allyl (1S, 2R, 3S)-3-[2-[Carbo-(1RS)-1-(2-methoxy-2-methylpropionyloxy)eth-1-yloxymethoxy]-4-methoxypheny]-1-(3,4-methlenedioxyphenyl)-5-prop-1-yloxy)indane-2-carboxylic acid sodium salt

Allyl (1S, 2R, 3S)-3-(2-carboxymethoxy-4-methoxyphenyl)-1-(3,4-methylenedioxyphenyl)-5-(prop-1-yloxy)indane-2-carboxylate (287 mg, 0.5 mmol) was dissolved in DMF (5 ml) and Cs2C03 (333 mg, 1 mmol) added followed by (1S)-1-bromoethyl 2-methoxy-2-methylpropionate (225 mg, 1 mmol). The reaction mixture was stirred at RT overnight, then partitioned between water and ethyl acetate, washed with dil. HCl and brine, dried (MgSO₄ anhyd.), filtered and evaporated to give an oil. The product was purified by column chromatography to provide the title compound (260 mg, 74 %) as a colorless oil.

### f) (1S,2R,3S)-3-[2-[Carbo-(1RS)-1-(2-methoxy-2-methylpropionyloxy)eth-1-yloxymethoxyl-4-methoxyphenyl]-1-(3,4-methylenedioxyphenyl)-5-prop-1-yloxy)indane-2-carboxylic acid sodium salt

Allyl (1S,2R,3S)-3-[2-Carbo-(1RS)-1-(2-methoxy-2-methylpropionyloxyeth-1-yloxymethoxy]-4-methoxyphenyl)-1-(3,4-methylenedioxyphenyl)-5-(prop-1-yloxy)indane-2-carboxylate (260 mg, 0.37 mmol) was dissolved in CH₂Cl₂ (2 ml) and tetrakis(triphenylphosphine)palladium(0) (36 mg, 0.037 mmol) added followed by tri-*n*-butyltin hydride (0.11 ml, 0.4 mol). The reaction mixture was stirred at RT for 3 h then quenched with 3N HCl and stirred for 20 min. The organic layer was diluted with ethyl acetate washed with water then brine, dried (MgSO₄ anhyd.), filtered and evaporated to give an oil. The product was purified by column chromatography to provide the title compound (210 mg, 85 %) as a colorless oil.
MS (exact mass) (M+Na)⁺·: 687.2415 (sodium salt)
(D = +0.3. mDa for C₃₆H₄₀O₁₂Na)

By the methods given above, the following compounds were made.

### (1RS,2SR,3RS)-3-[2-[(3-Carboxypyridin-2-yl)oxy]-4-methoxyphenyl]-1(3,4-methylenedioxyphenyl)-5-(prop-1-yloxy)indane-2-carboxylic acid.

m.p. 152-155°C

### (1RS,2SR,3RS)-3-[2-[Carbo(N,N-diethylcarbamoyl)methoxylmethoxy-4-methoxyphenyl]-1-(3,4-methylenedioxyphenyl)-5-(prop-1-yloxy)indane-2-carboxylic acid.

m.p. 181-182°C;

### Trans, Trans-1,3-Bis(4-methoxyphenyl)indane-2-carboxamide.

m.p. 223-225°C;

### (1RS,2SR,3SR)-3-[4-Methoxy-2-[2-(methylphosphinyl) eth-1-yl]phenyl]-1-(3,4-methylenedioxyphenyl)-5-(prop-1-yloxy)indane-2-carboxylic acid disodium salt:

(exact mass) M⁺+Na: 619.1462 (Δ = -1.2 mDa for C₃₀H₃₁O₈PNa₃)

### EXAMPLES 6-30

### Indan-5-yl (1RS,2SR,3RS)-3-(2-carboxymethoxy-4-methoxyphenyl)-1-(3,4-methylenedioxyphenyl)-5-(prop-1-yloxy)indane-2-carboxylate sodium salt.

m.p. 181-183°C dec.

### 3,5-Dimethoxyphenyl (1RS,2SR,3RS)-3-(2-carboxymethoxy-4-methoxyphenyl)-1-(3,4-methylenedioxyphenyl)-5-(prop-1-yloxy)indane-2-carboxylate sodium salt

m.p, 185-189°C dec.

### (1RS)-1-(2-Methoxy-2-methylpropionyloxy)eth-1-yl (1RS,2SR,3RS)-3-(2-carboxymethoxy-4-methoxyphenyl)-1-(3,4-methylenedioxyphenyl)-5-(prop-1-yloxy)indane-2-carboxylate sodium salt

m.p. 178-181°C dec.

### N,N-Dimethylcarbamoylmethyl (1RS,2SR,3RS)-3-(2-carboxymethoxy-4-methoxyphenyl)-1-(3,4-methylenedioxyphenyl)-5-(prop-1-yloxy)indane-2-carboxylate sodium salt

m.p. 170-174°C dec.

### Ethoxycarboxymethyl (1RS,2SR,3RS)-3-(2-carboxymethoxy-4-methoxyphenyl)-1-(3,4-methylenedioxyphenyl)-5-(prop-1-yloxy)indane-2-carboxylate sodium salt

MS (exact mass) (M+Na)⁺·: 645.1959 (sodium salt)
(D = -1.1 mDa for C₃₃H₃₄O₁₂Na)

### Benzoyloxymethyl (1RS,2SR,3RS)-3-(2-carboxymethoxy-4-methoxyphenyl)-1-(3,4-methylenedioxyphenyl)-5-(prop-1-yloxy)indane-2-carboxylate sodium salt

MS m/e : 672 (M+NH₄)⁺

### Cyclohexyloxycarboxymethyl (1RS,2SR,3RS)-3-(2-carboxymethoxy-4-methoxyphenyl)-1-(3,4-methylenedioxyphenyl)-5-(prop-1-yloxy)indane-2-carboxylate sodium salt

MS (exact mass) (M+Na)⁺·: 699.2453 (sodium salt)
(D = -3.5. mDa for C₃₇H₄₀O₁₂Na)

### Ethyl (1RS,2SR,3RS)-3-(2-carboxymethoxy-4-methoxyphenyl)-1-(3,4-methylenedioxyphenyl)-5-(prop-1-yloxy)indane-2-carboxylate sodium salt

MS (exact mass) M⁺·: 548.2040 (free acid)
(D = +0.6. mDa for C₃₁H₃₂O₉)

### (1S,2R,3S)-3-[2-[Carbo-(2',6'-dimethylphenoxy)methoxy]-4-methoxyphenyl]-1-(3,4-methylenedioxyphenyl)-5-(prop-1-yloxy)indane-2-carboxylic acid sodium salt

MS (exact mass) (M+Na)⁺·: 647.2264 (sodium salt)
D = -0.7. mDa for C₃₇H₃₆O₉Na)

### (1S,2R,3S)-3-[2-(Carbocyclopentyloxymethoxy)-4-methoxyphenyl]-1-(3,4-methylenedioxvphenyl)-5-(prop-1-yloxy)indane-2-carboxylic acid sodium salt

MS (exact mass) (M+Na)⁺·: 611.2282 (sodium salt)
(D = -2.5. mDa for C₃₄H₃₆O₉Na)

### (1S,2R,3S)-3-[2-[Carbo(indan-5-yloxy)methoxy]-4-methoxyphenyl]-1-(3,4-methylenedioxyphenyl)-5-(prop-1-yloxy)indane-2-carboxylic acid sodium salt

MS (exact mass) (M+Na)⁺·: 659.2281 (sodium salt)
(D = -2.4. mDa for C₃₈H₃₆O₉Na)

### (1S,2R,3S)-3-[2-Carbo(eth-1-yloxy)methoxy]-4-methoxyphenyl]-1-(3,4-methylenedioxyphenyl)-5-(prop-1-yloxy)indane-2-carboxylic acid sodium salt

MS (exact mass) (M+2Na-H)⁺·: 593.1769 (sodium salt)
(D = -0.6 mDa for C₃₁H₃₁O₉Na₂)

### (1RS,2SR,3RS)-3-(2-Carboethoxymethoxy-4-methoxyphenyl)-1-(3,4-methylenedioxyphenyl)-5-(prop-1-yloxy)indane-2-carboxylic acid

m.p. 148-149°C.

### (1RS,2SR,3RS)-2-Trifluoromethylsulfonamidomethyl-3-(2-carboxymethoxy-4-methoxyphenyl)-1-(3,4-methylenedioxyphenyl)-5-(prop-1-yloxy)indane

m.p. 184-186°C

### Indan-5-yl (1S,2R,3S)-3-(2-carboxymethoxy-4-methoxyphenyl)-1-(3,4-methylenedioxyphenyl)-5-(prop-1-yloxy)indane-2-carboxylate sodium salt

### Cyclopentyl (1S,2R,3S)-3-(2-carboxymethoxy-4-methoxyphenyl)-1-(3,4-methylenedioxyphenyl)-5-(prop-1-yloxy)indane-2-carboxylate sodium salt.

### Ethoxycarboxymethyl (1S,2R,3S)-3-(2-carboxymethoxy-4-methoxyphenyl)-1-(3,4-methylenedioxyphenyl)-5-(prop-1-yloxy)indane-2-carboxylate sodium salt.

### (1RS)-1-(1-Methylethoxycarboxy)eth-1-yl (1RS,2SR,3RS)-3-(2-carboxymethoxy-4-methoxyphenyl)-1-(3,4-methylenedioxyphenyl)-5-(prop-1-yloxy)indane-2-carboxylate sodium salt.

m.p. 151-155°C.

### Ethyl (1S,2R,3S)-3-(2-carboxymethoxy-4-methoxyphenyl)-1-(3,4-methylenedioxyphenyl)-5-(prop-1-yloxy)indane-2-carboxylate sodium salt

### (1S,2R,3S)-3-[2-Carbomethoxymethoxy-4-methoxyphenyl]-1-(3,4-methylenedioxyphenyl)-5-(prop-1-yloxy)indane-2-carboxylic acid sodium salt

### (1S,2R,3S)-3-[2-[Carbobenzoyloxymethoxymethoxy]-4-methoxyphenyl]-1-(3,4-methylenedioxyphenyl)-5-(prop-1-yloxy)indane-2-carboxylic acid sodium salt

### (1S,2R,3S)-3-[2-(Carboethoxycarboxyloxymethoxymethoxy)-4-methoxyphenyl]-1-(3,4-methylenedioxyphenyl)-5-(prop-1-yloxy)indane-2-carboxylic acid sodium salt

### (1S,2R,3S)-3-[2-(Carboacetoxymethoxymethoxy)-4-methoxyphenyl]-1-(3,4-methylenedioxyphenyl)-5-(prop-1-yloxy)indane-2-carboxylic acid sodium salt

### (1S,2R,3S)-3-[2-(Carbophthalidylmethoxy)-4-methoxyphenyl]-1-(3,4-methylenedioxyphenyl)-5-(prop-1-yloxy)indane-2-carboxylic acid sodium salt

### (1S,2R,3S)-3-[2-Carbo-(2-methoxy-2-methylpropionyloxymethoxymethoxy]-4-methoxyphenyl]-1-(3,4-methylenedioxyphenyl)-5-(prop-1-yloxy)indane-2-carboxylic acid sodium salt

### EXAMPLE 31

### (1S,2R,3S)-3-[2-Carbo-(2,2-dimethylpropanoyloxymethoxymethoxy]-4-methoxyphenyl]-1-(3,4-methylenedioxyphenyl)-5-(prop-1-yloxy)indane-2-carboxylic acid sodium salt

To a solution of (1S,2R,3S)-3-[2-Carboxymethoxy-4-methoxyphenyl]-1-(3,4-methylenedioxyphenyl)-5-(prop-1-yloxy)indane-2-carboxylic acid monopotassium salt (170 mg, 0.3 mmol) (obtained by treatment of the corresponding diacid (156 mg, 0.3 mmol) with 1 equiv. of KHCO₃ (30 mg, 0.3 mmol)) in DMF (4 mL) pivaloyloxymethyl iodide (74 mg, 0.3 mmol) was added. The reaction mixture was stirred at RT for 0.5 h and more pivaloyloxymethyl iodide was then added (20 mg. 0.08 mmol) and then stirred for an additional 0.5 h. The reaction mixture was partitioned between dilute aqueous HCl and ethyl acetate. The ethyl acetate extract was washed with water and brine and dried (MgSO₄ anhydrous). The solvent was removed *in vacuo* and the residue was purified by flash column chromatography (silica gel, Ethyl Acetate/hexane/HOAc 30/65/5) to obtain the desired compound as a white foam (140 mg, 73 %) as the free acid which was converted to its sodium salt. m.p. 128 - 133°C.
MS (exact mass) M⁺+Na : 679.2116 (sodium salt)
(D = +1.2 mDa for C₃₅H₃₇O₁₁Na₂)

### EXAMPLE 32

Formulations for pharmaceutical use incorporating compounds of the present invention can be prepared in various forms and with numerous excipients. Examples of such formulations are given below.

### Inhalant Formulation

A compound of Formula I, (1 mg to 100 mg) is aerosolized from a metered dose inhaler to deliver the desired amount of drug per use.

| Tablets/Ingredients | Per Tablet |
|---|---|
| 1. Active ingredient (Cpd of Form. I) | 40 mg |
| | |
| 2. Corn Starch | 20 mg |
| | |
| 3. Alginic acid | 20 mg |
| | |
| 4. Sodium Alginate | 20 mg |
| | |
| 5. Mg stearate | 1.3 mg |
| | 2.3 mg |

### Procedure for tablets:

- Step 1: Blend ingredients No. 1, No. 2, No. 3 and No. 4 in a suitable mixer/blender.
- Step 2: Add sufficient water portion-wise to the blend from Step 1 with careful mixing after each addition. Such additions of water and mixing until the mass is of a consistency to permit its conversion to wet granules.
- Step 3: The wet mass is converted to granules by passing it through an oscillating granulator using a No. 8 mesh (2.38 mm) screen.
- Step 4: The wet granules are then dried in an oven at 140°F (60°C) until dry.
- Step 5: The dry granules are lubricated with ingredient No. 5.
- Step 6: The lubricated granules are compressed on a suitable tablet press.

### Parenteral Formulation

A pharmaceutical composition for parenteral administration is prepared by dissolving an appropriate amount of a compound of formula I in polyethylene glycol with heating. This solution is then diluted with water for injections Ph Eur. (to 100 ml). The solution is then steriled by filtration through a 0.22 micron membrane filter and sealed in sterile containers.

## Claims

1. A compound of Formula (I): 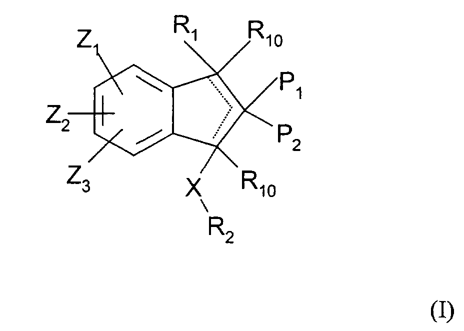 wherein:
R₁ is -X(CH₂)ₙAr or -X(CH₂)ₙR₈ or 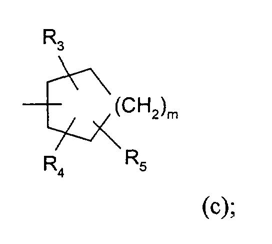
R₂ is hydrogen, Ar, C₁₋₄alkyl or (c);
P₁ is -X(CH₂)ₙR₈;
P₂ is -X(CH₂)ₙR₈ or -X-R₉-Y;
R₃ and R₅ are independently hydrogen, R₁₁, OH, C₁₋₈alkoxy, S(O)_{q}R₁₁, N(R₆)₂, Br, F, I, Cl, CF₃, NHCOR₆, R₁₁CO₂R₇, -X-R₉-Y or -X(CH₂)ₙR₈, wherein each methylene group within -X(CH₂)ₙR₈ may be unsubstituted or substituted by one or two -(CH₂)ₙAr groups;
R₄ is hydrogen, R₁₁, OH, C₁₋₅alkoxy, S(O)_{q}R₁₁, N(R₆)₂, -X(R₁₁), Br, F, I, Cl or NHCOR₆, wherein the C₁₋₅alkoxy may be unsubstituted or substituted by OH, methoxy or halogen;
R₆ is independently hydrogen or C₁₋₄alkyl;
R₇ is independently hydrogen, C₁₋₁₀alkyl, C₂₋₁₀alkenyl or C₂₋₈ alkynyl, all of which may be unsubstituted or substituted by one or more OH, N(R₆)₂, CO₂R₁₂, halogen or XC₁₋₅alkyl; or R₇ is (CH₂)ₙAr;
R₈ is hydrogen, R₁₁, CO₂R₇, CO₂C(R₁₁)₂O(CO)XR₇, PO₃(R₇)₂, SO₂NR₇R₁₁, NR₇SO₂R₁₁, CONR₇SO₂R₁₁, SO₃R₇, SO₂R₇, P(O)(OR₇)R₇, CN, C(O)N(R₆)₂, -CO₂(CH₂)ₘC(O)N(R₆)₂, C(R₁₁)₂N(R₇)₂, tetrazole or OR₆;
R₉ is (CH₂)ₙ, divalent C₁₋₁₀alkyl, divalent C₂₋₁₀alkenyl or phenyl, all of which may be unsubstituted or substituted by one or more OH, N(R₆)₂, COOH, halogen, or R₉ may be 〉C=O or XC₁₋₅alkyl;
R₁₀ is R₃ or R₄;
R₁₁ is hydrogen, Ar, C₁₋₈alkyl, C₂₋₈alkenyl, C₂₋₈alkynyl, all of which may be unsubstituted or substituted by one or more OH, CH₂OH, N(R₆)₂ or halogen; with the proviso that when R₁₁ is present in the group R₁₁CO₂R₇, R₁₁ is not hydrogen.
R₁₂ is hydrogen, C₁₋₆alkyl, C₂₋₆alkenyl or C₂₋₇alkynyl;
X is (CH₂)ₙ, O, NR₆ or S(O)_{q};
Y is CH₃ or X(CH₂)ₙAr;
Ar is: 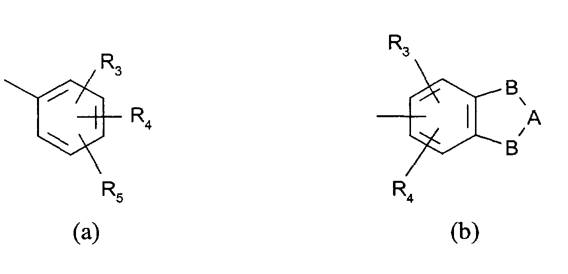
naphthyl, indolyl, pyridyl, thienyl, oxazolidinyl, oxazolyl, thiazolyl, isothiazolyl, pyrazolyl, triazolyl, tetrazolyl, imidazolyl, imidazolidinyl, thiazolidinyl, isoxazolyl, oxadiazolyl, thiadiazolyl, morpholinyl, piperidinyl, piperazinyl, pyrrolyl, or pyrimidyl; all of which may be unsubstituted or substituted by one or more R₃ or R₄ groups;
A is C=O or [C(R₆)₂]ₘ;
B is -CH₂- or -O-;
Z₁ and Z₂ are independently hydrogen, C₁₋₈alkyl, C₂₋₈alkenyl, C₂₋₈alkynyl, OH, C₁₋₈alkoxy, S(O)_{q}C₁₋₈alkyl, N(R₆)₂, Br, F, I, Cl, NHCOR₆, -X-R₉-Y, -X(CH₂)ₙR₈, phenyl, benzyl or C₃₋₆cycloalkyl wherein the C₁₋₈alkyl, C₂₋₈alkenyl or C₂₋₈alkynyl may be optionally substituted by COOH, OH,
B is -CH₂- or -O-;
Z₁ and Z₂ are independently hydrogen; C₁₋₈alkyl, C₂₋₈alkenyl, C₂₋₈alkynyl, OH, C₁₋₈alkoxy, S(O)_{q}C₁₋₈alkyl, N(R₆)₂, Br, F, I, Cl, NHCOR₆, -X(CH₂)ₙR₈, phenyl, benzyl or C₃₋₆cycloalkyl wherein the C₁₋₈alkyl, C₂₋₈alkenyl or C₂₋₈alkynyl may be optionally substituted by COOH, OH, CO(CH₂)ₙCH₃, CO(CH₂)ₙCH₂N(R₆)₂ or halogen; or Z₁ and Z₂ together may be -O-A-O- on contiguous carbons;
Z₃ is Z₁ or XR₉Y;
q is zero, one or two;
n is an integer from 0 to six; and
m is 1, 2 or 3; are excluded.

2. A compound according to claim 1 wherein
R₁ is X(CH₂)ₙAr, dihydrobenzofuranyl, benzodioxanyl, cyclohexyl or C₁₋ ₄alkyl;
R₂ is a moiety of formula (a) or (b), C₁₋₄alkyl, indolyl or hydrogen;
R₃ and R₅ are independently hydrogen, OH, C₁₋₅alkoxy, halogen, -OC₁₋ ₄alkyl phenyl, R₁₁CO₂R₇, C₁₋₄alkyl, N(R₆)₂, NH(CO)CH₃, -X(CH₂)ₙR₈, -X-R₉-Y, pyridyl, phenyl or S(O)_{q}C₁₋₅alkyl;
R₄ is hydrogen, OH, C₁₋₅alkoxy, halogen, C₁₋₄alkyl, N(R₆)₂, NH(CO)CH₃ or S(O)_{q}C₁₋₅alkyl;
Z₁, Z₂ and Z₃ are independently X-R₉-Y, benzyl, hydrogen, OH, C₁₋ ₅alkoxy, -N(R₆)₂, S(O)_{q}C₁₋₈alkyl, NHCOR₆, X(CH₂)ₙR₈ or halogen, or Z₁ and Z₂ together may be -O-A-O- on contiguous carbons;
P₁ and P₂ are independently hydrogen, CO₂H or tetrazole;
Ar is a moiety of formula (a), or (b) or pyridyl; and
X is (CH₂)ₙ or oxygen.

3. A compound according to claim 1 or 2 wherein:
R₃ is hydrogen, -X(CH₂)ₙR₈ or R₁₁CO₂R₇;
R₄ and R₅ are independently hydrogen, OH, C₁₋₅alkoxy, SC₁₋₅alkyl, F, Br, C₁₋₃alkyl or NH₂; and
Z₁ and Z₃ are hydrogen and Z₂ is hydrogen, OH, C₁₋₅alkoxy, halogen, X(CH₂)ₙR₈, NH₂, benzyl or NH(CO)CH₃, or Z₁ and Z₂ together may be -O-A-O-on contiguous carbons.

4. A compound according to claim 1, 2 or 3 wherein
R₁ is a moiety of formula (b);
A is CH₂, B is -O-;
there is no optional double bond;
R₁ and XR₂ are trans to P₁;
Z₂ is OH, C₁₋₅alkoxy, -OCH₂CH₂CH₃ or hydrogen;
Z₁ is hydrogen;
R₃ is hydrogen, XAr, X(CH₂)_{q}CO₂H, X(CH₂)_{q}CONR₇SO₂R₁₁, or CH=CHCO₂H;
R₄ is hydrogen or C₁₋₂alkoxy; and
R₅, R₁₀ and P₂ are hydrogen.

5. A compound which is (+)(1S, 2R, 3S)-3-(2-carboxymethoxy-4-methoxyphenyl)-1-(3,4-methylenedioxyphenyl)-5-(prop-1-yloxy)indane-2-carboxylic acid, or a pharmaceutically acceptable salt thereof.

6. A compound which is (+)(1S, 2R, 3S)-3-[2-(2-hydroxyeth-1-yloxy)-4-methoxyphenyl]-1-(3,4-methylenedioxyphenyl)-5-(prop- 1-yloxy)indane-2-carboxylic acid, or a pharmaceutically acceptable salt thereof.

7. A compound which is (+)(1S, 2R, 3S)-3-[2-(2-hydroxyeth-1-yloxy)-4-methoxyphenyl]-1-(3,4-methylenedioxyphenyl)-5-(prop-1-yloxy)indane-2-carboxylic acid, disodium salt.

8. A compound which is (+)(1S, 2R, 3S)-3-[2-(2-hydroxyeth-1-yloxy)-4-methoxyphenyl]-1-(3,4-methylenedioxyphenyl)-5-(prop-1-yloxy)indane-2-carboxylic acid, dicyclohexylamine salt.

9. A compound which is (1RS, 2SR, 3RS)-3-[2-[(4-carboxypyridin-3-yl)oxy]-4- methoxyphenyl]-1-(3,4-methylenedioxyphenyl)-5-(prop-1-yloxy)indane-2-carboxylic acid, or a pharmaceutically acceptable salt thereof.

10. A pharmaceutical composition comprising a compound according to any of claims 1 to 9 or a pharmaceutically acceptable salt thereof and a pharmaceutically acceptable carrier.

11. Use of a compound according to any of claims 1 to 9 or a pharmaceutically acceptable salt thereof in the manufacture of a medicament for the treatment of a condition requiring antagonizing endothelin receptors.

12. Use of a compound according to any of claims 1 to 9 or a pharmaceutically acceptable salt thereof in the manufacture of a medicament for the treatment of hypertension, renal failure or cerebrovascular disease.

13. A process for the preparation of a compound of formula (I) as defined in any of claims 1 to 9 or a pharmaceutically acceptable salt thereof, which process comprises reacting a compound of formula (11): 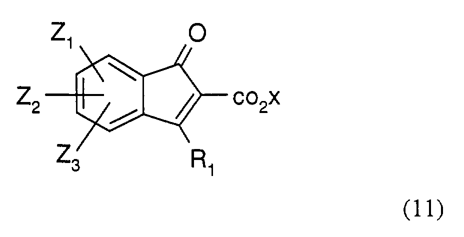 wherein Z₁, Z₂, Z₃ and R₁ are as defined in claim 1 or groups convertable thereto, and X is C₁₋₅alkyl, with an organomagnesium compound of formula (12):
R₂-(CH₂)ₙ-MgBr (12)
wherein R₂ is as defined in claim 1 or a group convertable thereto, in a suitable solvent to provide a compound of formula (13): 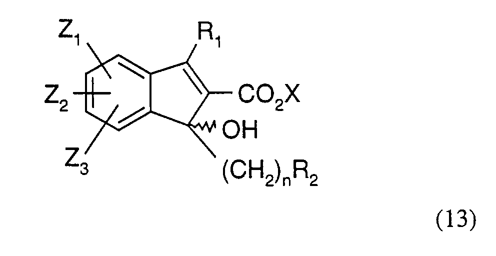 which is reduced and thereafter, when desired or necessary undergoes:
a) insertion of R₁₀ (when other than hydrogen) through conjugate additon; and/or
b) alkylation or acylation to give compounds wherein P₁ and P₂ are other than CO₂H; and/or
c) conversion of groups R₁, R₂, Z₁, Z₂ and Z₃ to other groups R₁, R₂, Z₁, Z₂ and Z₃;
to afford a compound of formula (I).

## Patentansprüche

1. Verbindung der Formel (I): 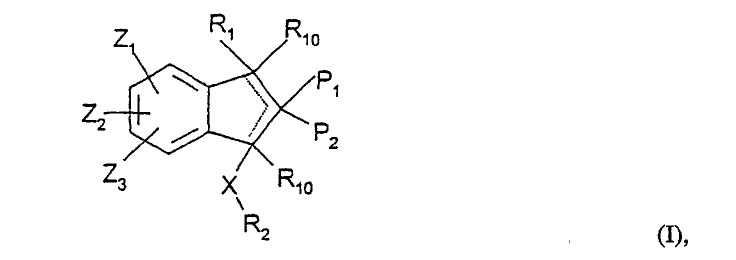 in der:
R₁ einen Rest der Formel -X(CH₂)ₙAr oder -X(CH₂)ₙR₈ oder 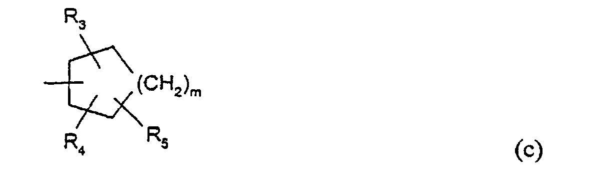 bedeutet;
R₂ ein Wasserstoffatom, einen Ar-, C₁₋₄-Alkylrest oder (c) darstellt;
P₁ einen Rest der Formel -X(CH₂)ₙR₈ bedeutet;
P₂ einen Rest der Formel -X(CH₂)ₙR₈ oder -X-R₉-Y darstellt;
R₃ und R₅ unabhängig voneinander ein Wasserstoffatom, den Rest R₁₁, eine OH-Gruppe, einen C₁₋₈-Alkoxyrest, einen Rest der Formel S(O)_{q}R₁₁, N(R₆)₂, ein Brom-, Fluor-, Iod-, Chloratom, eine CF₃-Gruppe, einen Rest der Formel NHCOR₆, R₁₁CO₂R₇, -X-R₉-Y oder -X(CH₂)ₙR₈ bedeuten, wobei jede Methylengruppe in dem Rest der Formel -X(CH₂)ₙR₈ unsubstituiert oder mit einem oder zwei Resten der Formel -(CH₂)ₙAr substituiert sein kann;
R₄ ein Wasserstoffatom, den Rest R₁₁, eine OH-Gruppe, einen C₁₋₅-Alkoxyrest, einen Rest der Formel S(O)_{q}R₁₁, N(R₆)₂, -X(R₁₁), ein Brom-, Fluor-, Iod-, Chloratom oder einen Rest der Formel NHCOR₆ darstellt, wobei der C₁₋₅-Alkoxyrest unsubstituiert oder mit einer OH-, Methoxygruppe oder einem Halogenatom substituiert sein kann;
R₆ unabhängig ein Wasserstoffatom oder einen C₁₋₄-Alkylrest bedeutet;
R₇ unabhängig ein Wasserstoffatom, einen C₁₋₁₀-Alkyl-, C₂₋₁₀-Alkenyl- oder C₂₋₈-Alkinylrest darstellt, die alle unsubstituiert oder mit einer oder mehreren OH-Gruppen, Resten der Formel N(R₆)₂, CO₂R₁₂, Halogenatomen oder XC₁₋₅-Alkylresten substituiert sein können; oder R₇ einen Rest der Formel (CH₂)ₙAr bedeutet;
R₈ ein Wasserstoffatom, den Rest R₁₁, einen Rest der Formel CO₂R₇, CO₂C(R₁₁)₂O-(CO)XR₇, PO₃(R₇)₂, SO₂NR₇R₁₁, NR₇SO₂R₁₁, CONR₇SO₂R₁₁, SO₃R₇, SO₂R₇, P(O)(OR₇)R₇, eine CN-Gruppe, einen Rest der Formel C(O)N(R₆)₂, -CO₂(CH₂)ₘC(O)N(R₆)₂, C(R₁₁)₂N(R₇)₂, eine Tetrazolgruppe oder einen Rest der Formel OR₆ darstellt;
R₉ einen Rest der Formel (CH₂)ₙ, einen zweiwertigen C₁₋₁₀-Alkyl-, zweiwertigen C₂₋₁₀-Alkenylrest oder eine Phenylgruppe bedeutet, die alle unsubstituiert oder mit einer oder mehreren OH-Gruppen, Resten der Formel N(R₆)₂, COOH-Gruppen, Halogenatomen substituiert sein können; oder R₉ eine >C=0-Gruppe oder einen XC₁₋₅-Alkylrest darstellen kann;
R₁₀ den Rest R₃ oder R₄ bedeutet;
R₁₁ ein Wasserstoffatom, einen Ar-, C₁₋₈-Alkyl-, C₂₋₈-Alkenyl- oder C₂₋₈-Alkinylrest darstellt, die alle unsubstituiert oder mit einer oder mehreren OH-, CH₂OH-Gruppen, Resten der Formel N(R₆)₂ oder Halogenatomen substituiert sein können; mit der Maßgabe, dass, wenn R₁₁ in dem Rest der Formel R₁₁CO₂R₇ vorliegt, R₁₁ kein Wasserstoffatom bedeutet;
R₁₂ ein Wasserstoffatom, einen C₁₋₆-Alkyl-, C₂₋₆-Alkenyl- oder C₂₋₇-Alkinylrest darstellt;
X einen Rest der Formel (CH₂)ₙ, ein Sauerstoffatom, einen Rest der Formel NR₆ oder S(O)_{q} bedeutet;
Y eine CH₃-Gruppe oder einen Rest der Formel X(CH₂)ₙAr darstellt;
Ar einen Rest der Formel: 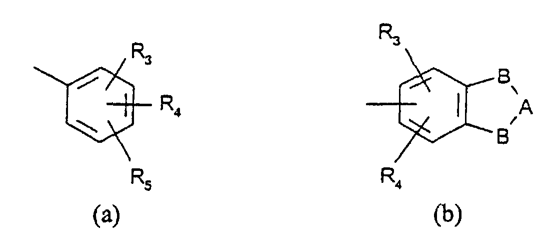
eine Naphthyl-, Indolyl-, Pyridyl-, Thienyl-, Oxazolidinyl-, Oxazolyl-, Thiazolyl-, Isothiazolyl-, Pyrazolyl-, Triazolyl-, Tetrazolyl-, Imidazolyl-, Imidazolidinyl-, Thiazolidinyl-, Isoxazolyl-, Oxadiazolyl-, Thiadiazolyl-, Morpholinyl-, Piperidinyl-, Piperazinyl-, Pyrrolyl- oder Pyrimidylgruppe bedeutet, die alle unsubstituiert oder mit einem oder mehreren Resten R₃ oder R₄ substituiert sein können;
A eine C=O-Gruppe oder einen Rest der Formel [C(R₆)₂]ₘ darstellt;
B eine -CH₂- oder -O-Gruppe bedeutet;
Z₁ und Z₂ unabhängig voneinander ein Wasserstoffatom, einen C₁₋₈-Alkyl-, C₂₋₈-Alkenyl-, C₂₋₈-Alkinylrest, eine OH-Gruppe, einen C₁₋₈-Alkoxy-, S(O)_{q}C₁₋₈-Alkylrest, einen Rest der Formel N(R₆)₂, ein Brom-, Fluor-, Iod-, Chloratom, einen Rest der Formel NHCOR₆, -X-R₉-Y, -X(CH₂)ₙR₈, eine Phenyl-, Benzylgruppe oder einen C₃₋₆-Cycloalkylrest darstellen, wobei der C₁₋₈-Alkyl-, C₂₋₈-Alkenyl- oder C₂₋₈-Alkinylrest gegebenenfalls mit einer COOH-, OH-Gruppe, einem Rest der Formel CO(CH₂)ₙCH₃, CO(CH₂)ₙCH₂N(R₆)₂ oder einem Halogenatom substituiert sein kann; oder Z₁ und Z₂ zusammen einen Rest der Formel-O-A-O- an benachbarten Kohlenstoffatomen bedeuten können;
Z₃ den Rest Z₁ oder einen Rest der Formel -X-R₉-Y darstellt;
q null, eins oder zwei ist;
n eine ganze Zahl von 0 bis sechs ist;
m 1, 2 oder 3 ist; und die punktierte Linie die mögliche Anwesenheit einer Doppelbindung anzeigt; oder ein pharmazeutisch verträgliches Salz davon; mit der Maßgabe, dass
• R₂ kein Wasserstoffatom bedeutet, wenn X einen Rest der Formel S(O)_{q} darstellt;
• R₁₁ und R₇ kein Wasserstoffatom bedeuten, wenn q in dem Rest der Formel S(O)_{q}R₁₁ oder S(O)_{q}R₇ 1 oder 2 ist;
• wenn die gegebenenfalls vorhandene Doppelbindung vorliegt, es nur einen Rest R₁₀ und keinen Rest P₁ gibt, und P₂ keinen Rest der Formel NR₆R₉Y darstellt;
• wenn die gegebenenfalls vorhandene Doppelbindung in Formel (I) vorliegt, und der Rest der Formel X-R₂ an die Doppelbindung gebunden ist, X keinen Rest der Formel NR₆ bedeutet;
• wenn die gegebenenfalls vorhandene Doppelbindung vorliegt, und der Rest R₁ direkt an die Doppelbindung gebunden ist, R₁ keinen Rest der Formel NR₆Ar darstellt;
• wenn der Rest R₃, R₅, Z₁, Z₂ oder Z₃ einen Rest der Formel X(CH₂)ₙR₈ bedeutet, und n nicht 0 ist, X ein Sauerstoffatom oder einen Rest der Formel NR₆ darstellt, wenn R₈ einen Rest der Formel OR₆ oder eine CO₂H-Gruppe bedeutet;
• wenn R₈ einen Rest der Formel CO₂C(R₁₁)₂O(CO)XR₇ darstellt, X keinen Rest der Formel S(O)_{q} bedeutet;
• die Verbindung der Formel (I) kein 1,3-Diphenylinden-2-carbonsäuremethylester, 1,3-Diphenylinden-2-carbonsäureethylester, 1,3-Diphenyl-2-cyanoinden oder 1,3-Diphenyl-3-hydroxyindan-2-carbonsäureethylester ist;
und ferner mit der Maßgabe, dass Verbindungen, in denen:
R₁ einen Rest der Formel -X(CH₂)ₙAr oder -X(CH₂)ₙR₈ oder 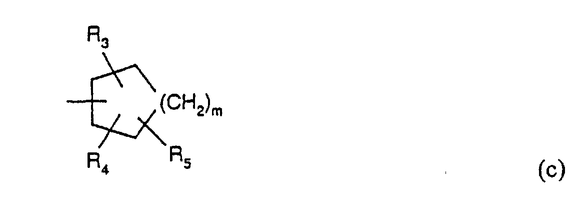 darstellt;
R₂ ein Wasserstoffatom, einen Ar-Rest oder (c) bedeutet;
P₁ einen Rest der Formel -X(CH₂)ₙR₈ darstellt;
P₂ einen Rest der Formel -X(CH₂)ₙR₈ oder -XR₉-Y bedeutet;
R₃ und R₅ unabhängig voneinander ein Wasserstoffatom, den Rest R₁₁, eine OH-Gruppe, einen C₁₋₈-Alkoxyrest, einen Rest der Formel S(O)_{q}R₁₁, N(R₆)₂, ein Brom-, Fluor-, Iod-, Chloratom, eine CF₃-Gruppe, einen Rest der Formel NHCOR₆, -XR₉-Y oder -X(CH₂)ₙ-R₈ darstellen, wobei die Methylengruppen des Restes der Formel -X(CH₂)ₙR₈ unsubstituiert oder mit einem oder mehreren Resten der Formel -(CH₂)ₙAr substituiert sein können;
R₄ ein Wasserstoffatom, den Rest R₁₁, eine OH-Gruppe, einen C₁₋₅-Alkoxyrest, einen Rest der Formel S(O)_{q}R₁₁, N(R₆)₂, -X(R₁₁), ein Brom-, Fluor-, Iod-, Chloratom oder einen Rest der Formel NHCOR₆ bedeutet, wobei der C₁₋₅-Alkoxyrest unsubstituiert oder mit einer OH-, Methoxygruppe oder einem Halogenatom substituiert sein kann;
R₆ unabhängig ein Wasserstoffatom oder einen C₁₋₄-Alkylrest darstellt;
R₇ unabhängig ein Wasserstoffatom, einen C₁₋₆-Alkylrest oder einen Rest der Formel (CH₂)ₙAr bedeutet;
R₈ ein Wasserstoffatom, den Rest R₁₁, eine CO₂H-, PO₃H₂-Gruppe, einen Rest der Formel P(O)(OH)R₇ oder eine Tetrazolgruppe darstellt;
R₉ einen C₁₋₁₀-Alkyl-, C₂₋₁₀-Alkenylrest oder eine Phenylgruppe bedeutet, die alle unsubstituiert oder mit einer oder mehreren OH-Gruppen, Resten der Formel N(R₆)₂, COOH-Gruppen, Halogenatomen oder XC₁₋₅-Alkylresten substituiert sein können;
R₁₀ den Rest R₃ oder R₄ darstellt;
R₁₁ einen C₁₋₈-Alkyl-, C₂₋₈-Alkenyl- oder C₂₋₈-Alkinylrest bedeutet, die alle unsubstituiert oder mit einer oder mehreren OH-, CH₂OH-Gruppen, Resten der Formel N(R₆)₂ oder Halogenatomen substituiert sein können;
X einen Rest der Formel (CH₂)ₙ, ein Sauerstoffatom, einen Rest der Formel NR₆ oder S(O)_{q} darstellt;
Y eine CH₃-Gruppe oder einen Rest der Formel -CH₂X(CH₂)ₙAr bedeutet;
Ar einen Rest der Formel: 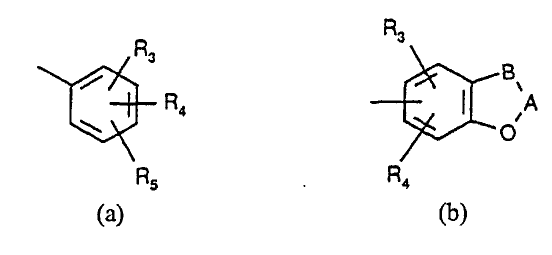
eine Naphthyl-, Indolyl-, Pyridyl- oder Thienyl-, Oxazolidinyl-, Oxazolyl-, Thiazolyl-, Isothiazolyl-, Pyrazolyl-, Triazolyl-, Tetrazolyl-, Imidazolyl-, Imidazolidinyl-, Thiazolidinyl-, Isoxazolyl-, Oxadiazolyl-, Thiadiazolyl-, Morpholinyl-, Piperidinyl-, Piperazinyl-, Pyrrolyl- oder Pyrimidylgruppe darstellt, die alle unsubstituiert oder mit einem oder mehreren Resten R₃ oder R₄ substituiert sein können;
A eine C=O-Gruppe oder einen Rest der Formel [C(R₆)₂]ₘ bedeutet;
B eine -CH₂- oder -O-Gruppe darstellt;
Z₁ und Z₂ unabhängig voneinander ein Wasserstoffatom, einen C₁₋₈-Alkyl-, C₂₋₈-Alkenyl-, C₂₋₈-Alkinylrest, eine OH-Gruppe, einen C₁₋₈-Alkoxy-, S(O)_{q}C₁₋₈-Alkylrest, einen Rest der Formel N(R₆)₂, ein Brom-, Fluor-, Iod-, Chloratom, einen Rest der Formel NHCOR₆, -X(CH₂)ₙR₈, eine Phenyl-, Benzylgruppe oder einen C₃₋₆-Cycloalkylrest bedeuten, wobei der C₁₋₈-Alkyl-, C₂₋₈-Alkenyl- oder C₂₋₈-Alkinylrest gegebenenfalls mit einer COOH-, OH-Gruppe, einem Rest der Formel CO(CH₂)ₙCH₃, CO(CH₂)ₙCH₂N(R₆)₂ oder einem Halogenatom substituiert sein kann; oder Z₁ und Z₂ zusammen einen Rest der Formel -O-A-O- an benachbarten Kohlenstoffatomen darstellen können;
Z₃ den Rest Z₁ oder einen Rest der Formel XR₉Y bedeutet;
q null, eins oder zwei ist;
n eine ganze Zahl von 0 bis sechs ist; und
m 1, 2 oder 3 ist;
ausgeschlossen sind.

2. Verbindung nach Anspruch 1, wobei:
R₁ einen Rest der Formel X(CH₂)ₙAr, eine Dihydrobenzofuranyl-, Benzodioxanyl-, Cyclohexylgruppe oder einen C₁₋₄-Alkylrest darstellt;
R₂ eine Einheit der Formel (a) oder (b), einen C₁₋₄-Alkylrest, eine Indolylgruppe oder ein Wasserstoffatom bedeutet;
R₃ und R₅ unabhängig voneinander ein Wasserstoffatom, eine OH-Gruppe, einen C₁₋₅-Alkoxyrest, ein Halogenatom, einen -OC₁₋₄-Alkylphenylrest, einen Rest der Formel R₁₁CO₂R₇, einen C₁₋₄-Alkylrest, einen Rest der Formel N(R₆)₂, NH(CO)CH₃, -X-(CH₂)ₙR₈, -X-R₉-Y, eine Pyridyl-, Phenylgruppe oder einen S(O)_{q}C₁₋₅-Alkylrest darstellen;
R₄ ein Wasserstoffatom, eine OH-Gruppe, einen C₁₋₅-Alkoxyrest, ein Halogenatom, einen C₁₋₄-Alkylrest, einen Rest der Formel N(R₆)₂, NH(CO)CH₃ oder einen S(O)_{q}C₁₋₅-Alkylrest bedeutet;
Z₁, Z₂ und Z₃ unabhängig voneinander einen Rest der Formel X-R₉-Y, eine Benzylgruppe, ein Wasserstoffatom, eine OH-Gruppe, einen C₁₋₅-Alkoxyrest, einen Rest der Formel -N(R₆)₂, einen S(O)_{q}C₁₋₈-Alkylrest, einen Rest der Formel NHCOR₆, X(CH₂)ₙR₈ oder ein Halogenatom darstellen; oder Z₁ und Z₂ zusammen einen Rest der Formel -O-A-O- an benachbarten Kohlenstoffatomen bedeuten können;
P₁ und P₂ unabhängig voneinander ein Wasserstoffatom, eine CO₂H- oder Tetrazolgruppe darstellen;
Ar eine Einheit der Formel (a) oder (b) oder eine Pyridylgruppe bedeutet; und
X einen Rest der Formel (CH₂)ₙ oder ein Sauerstoffatom darstellt.

3. Verbindung nach Anspruch 1 oder 2, wobei:
R₃ ein Wasserstoffatom, einen Rest der Formel -X(CH₂)ₙR₈ oder R₁₁CO₂R₇ bedeutet;
R₄ und R₅ unabhängig voneinander ein Wasserstoffatom, eine OH-Gruppe, einen C₁₋₅-Alkoxy-, SC₁₋₅-Alkylrest, ein Fluor-, Bromatom, einen C₁₋₃-Alkylrest oder eine NH₂-Gruppe darstellen; und
Z₁ und Z₃ ein Wasserstoffatom bedeuten, und Z₂ ein Wasserstoffatom, eine OH-Gruppe, einen C₁₋₅-Alkoxyrest, ein Halogenatom, einen Rest der Formel X(CH₂)ₙR₈, eine NH₂-, Benzyl- oder NH(CO)CH₃-Gruppe bedeuten, oder Z₁ und Z₂ zusammen einen Rest der Formel -O-A-O- an benachbarten Kohlenstoffatomen bedeuten können.

4. Verbindung nach Anspruch 1, 2 oder 3, wobei:
R₁ eine Einheit der Formel (b) darstellt;
A eine CH₂-Gruppe bedeutet, und B eine -O-Gruppe darstellt;
es keine gegebenenfalls vorhandene Doppelbindung gibt;
R₁ und XR₂ in trans-Stellung zu P₁ stehen;
Z₂ eine OH-Gruppe, einen C₁₋₅-Alkoxyrest, eine -OCH₂CH₂CH₃-Gruppe oder ein Wasserstoffatom bedeutet;
Z₁ ein Wasserstoffatom darstellt;
R₃ ein Wasserstoffatom, einen Rest der Formel XAr, X(CH₂)_{q}CO₂H, X(CH₂)_{q}CON-R₇SO₂R₁₁ oder eine CH=CHCO₂H-Gruppe bedeutet;
R₄ ein Wasserstoffatom oder einen C₁₋₂-Alkoxyrest darstellt; und
R₅, R₁₀ und P₂ ein Wasserstoffatom bedeuten.

5. Verbindung, nämlich (+)(1S,2R,3S)-3-(2-Carboxymethoxy-4-methoxyphenyl)-1-(3,4-methylendioxyphenyl)-5-(prop-1-yloxy)indan-2-carbonsäure oder ein pharmazeutisch verträgliches Salz davon.

6. Verbindung, nämlich (+)(1S,2R,3S)-3-[2-(2-Hydroxyeth-1-yloxy)-4-methoxyphenyl]-1-(3,4-methylendioxyphenyl)-5-(prop-1-yloxy)indan-2-carbonsäure oder ein pharmazeutisch verträgliches Salz davon.

7. Verbindung, nämlich das Dinatriumsalz der (+)(1S,2R,3S)-3-[2-(2-Hydroxyeth-1-yloxy)-4-methoxyphenyl]-1-(3,4-methylendioxyphenyl)-5-(prop-1-yloxy)indan-2-carbonsäure.

8. Verbindung, nämlich das Dicyclohexylaminsalz der (+)(1S,2R,3S)-3-[2-(2-Hydroxyeth-1-yloxy)-4-methoxyphenyl]-1-(3,4-methylendioxyphenyl)-5-(prop-1-yloxy)indan-2-carbonsäure.

9. Verbindung, nämlich (1RS,2SR,3RS)-3-[2-[(4-Carboxypyridin-3-yl)oxy]-4-methoxyphenyl]-1-(3,4-methylendioxyphenyl)-5-(prop-1-yloxy)indan-2-carbonsäure oder ein pharmazeutisch verträgliches Salz davon.

10. Arzneimittel, umfassend eine Verbindung nach einem der Ansprüche 1 bis 9 oder ein pharmazeutisch verträgliches Salz davon und einen pharmazeutisch verträglichen Träger.

11. Verwendung einer Verbindung nach einem der Ansprüche 1 bis 9 oder eines pharmazeutisch verträglichen Salzes davon bei der Herstellung eines Arzneimittels zur Behandlung einer Erkrankung, die einen Endothelinrezeptor-Antagonismus erfordert.

12. Verwendung einer Verbindung nach einem der Ansprüche 1 bis 9 oder eines pharmazeutisch verträglichen Salzes davon bei der Herstellung eines Arzneimittels zur Behandlung von Hypertonie, Nierenversagen oder einer zerebrovaskulären Erkrankung.

13. Verfahren zur Herstellung einer Verbindung der Formel (I) nach einem der Ansprüche 1 bis 9 oder eines pharmazeutisch verträglichen Salzes davon, das die Umsetzung einer Verbindung der Formel (11): 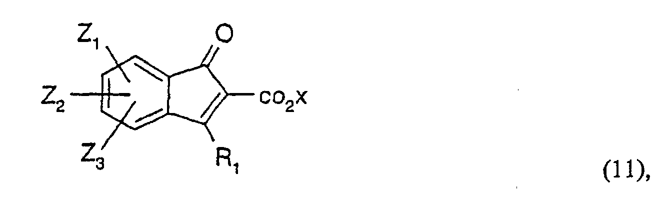 in der Z₁, Z₂, Z₃ und R₁ wie in Anspruch 1 definiert sind oder dazu umwandelbare Reste darstellen, und X einen C₁₋₅-Alkylrest bedeutet, mit einer Organomagnesiumverbindung der Formel (12):
R₂-(CH₂)ₙ-MgBr (12),
in der R₂ wie in Anspruch 1 definiert ist oder einen dazu umwandelbaren Rest darstellt, in einem geeigneten Lösungsmittel umfasst, wobei eine Verbindung der Formel (13): 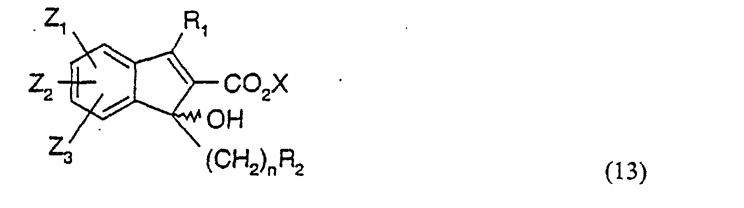 bereitgestellt wird, die reduziert wird und danach, falls erwünscht oder notwendig:
a) eine Einführung des Restes R₁₀ (wenn er kein Wasserstoffatom bedeutet) durch konjugierte Addition und/oder
b) eine Alkylierung oder Acylierung, wobei sich Verbindungen ergeben, in denen P₁ und P₂ keine CO₂H-Gruppe darstellen, und/oder
c) eine Umwandlung der Reste R₁, R₂, Z₁, Z₂ und Z₃ in andere Reste R₁, R₂, Z₁, Z₂ und Z₃ durchläuft, wobei sich eine Verbindung der Formel (I) ergibt.

## Revendications

1. Composé de formule (I) : 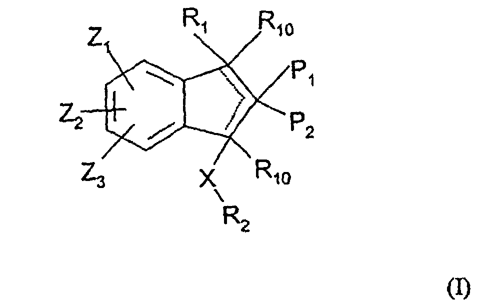 dans laquelle :
R₁ représente un groupe -X(CH₂)ₙAr ou -X(CH₂)ₙR₈ ou 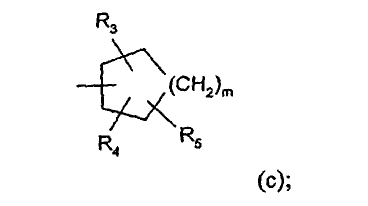
R₂ représente l'hydrogène, un groupe Ar, alkyle en C₁ à C₄ ou (c) ;
P₁ représente un groupe -X(CH₂)ₙR₈ ;
P₂ représente un groupe -X(CH₂)ₙR₈ ou -X-R₉-Y ;
R₃ et R₅ représentent, indépendamment, l'hydrogène, un groupe R₁₁, OH, alkoxy en C₁ à C₈, S(O)_{q}R₁₁, N(R₆)₂, Br, F, I, Cl, CF₃, NHCOR₆, R₁₁CO₂R₇, -X-R₉-Y ou -X(CH₂)ₙR₈, chaque groupe méthylène dans -X(CH₂)ₙR₈ pouvant être non substitué ou substitué avec un ou deux groupes -(CH₂)ₙAr ;
R₄ représente l'hydrogène, un groupe R₁₁, OH, alkoxy en C₁ à C₅, S(O)_{q}R₁₁, N(R₆)₂, -X(R₁₁), Br, F, I, Cl ou NCOR₆, le groupe alkoxy en C₁ à C₅ pouvant être non substitué ou substitué avec un groupe OH, méthoxy ou halogéno ;
R₆ réprésente, indépendamment, l'hydrogène ou un groupe alkyle en C₁ à C₄ ;
R₇ représente, indépendamment, l'hydrogène, un groupe alkyle en C₁ à C₁₀, alcényle en C₂ à C₁₀ ou alcynyle en C₂ à C₈, tous ces groupes pouvant être non substitués ou substitués avec un ou plusieurs groupes OH, N(R₆)₂, CO₂R₁₂, halogéno ou X(alkyle en C₁ à C₅) ; ou bien R₇ représente un groupe (CH₂)ₙAr ;
R₈ représente l'hydrogène, un groupe R₁₁, CO₂R₇, CO₂C(R₁₁)₂O(CO)XR₇, PO₃(R₇)₂, SO₂NR₇R₁₁, NR₇SO₂R₁₁, CONR₇SO₂R₁₁, SO₃R₇, SO₂R₇, P(O)(OR₇)R₇, CN, C(O)N(R₆)₂, -CO₂(CH₂)ₘC(O)N(R₆)₂, C(R₁₁)₂N(R₇)₂, tétrazole ou OR₆ ;
R₉ représente un groupe (CH₂)ₙ, alkyle en C₁ à C₁₀ divalent, alcényle en C₂ à C₁₀ divalent ou phényle, tous ces groupes pouvant être non substitués ou substitués avec un ou plusieurs groupes OH, N(R₆)₂, COOH, halogéno, ou bien R₉ peut représenter un groupe 〉C=O ou X(alkyle en C₁ à C₅) ;
R₁₀ représente R₃ ou R₄ ;
R₁₁ représente l'hydrogène, un groupe Ar, alkyle en C₁ à C₈, alcényle en C₂ à C₈ ou alcynyle en C₂ à C₈, tous ces groupes pouvant être non substitués ou substitués avec un ou plusieurs groupes OH, CH₂OH, N(R₆)₂ ou halogéno ; sous réserve que, lorsque R₁₁ est présent dans le groupe R₁₁CO₂R₇, R₁₁ ne représente pas l'hydrogène ;
R₁₂ représente l'hydrogène, un groupe alkyle en C₁ à C₆, alcényle en C₂ à C₆ ou alcynyle en C₂ à C₇ ;
X représente un groupe (CH₂)ₙ, O, NR₆ ou S(O)_{q} ;
Y représente un groupe CH₃ ou X(CH₂)ₙAr ;
Ar représente un groupe : 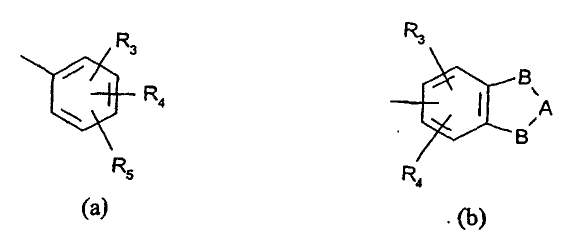
naphtyle, indolyle, pyridyle, thiényle, oxazolidinyle, oxazolyle, thiazolyle, isothiazolyle, pyrazolyle, triazolyle, tétrazolyle, imidazolyle, imidazolidinyle, thiazolidinyle, isoxazolyle, oxadiazolyle, thiadiazolyle, morpholinyle, pipéridinyle, pipérazinyle, pyrrolyle ou pyrimidiyle ; tous ces groupes pouvant être non substitués ou substitués avec un ou plusieurs groupes R₃ ou R₄ ;
A représente un groupe C=O ou [C(R₆)₂]ₘ ;
B représente un groupe -CH₂- ou -O- ;
Z₁ et Z₂ représentent, indépendamment, l'hydrogène, un groupe alkyle en C₁ à C₈, alcényle en C₂ à C₈, alcynyle en C₂ à C₈, OH, alkoxy en C₁ à C₈, S(O)_{q}(alkyle en C₁ à C₈), N(R₆)₂, Br, F, I, Cl, NHCOR₆, -X-R₉-Y, -X(CH₂)ₙR₈, phényle, benzyle ou cycloalkyle en C₃ à C₆, ledit groupe alkyle en C₁ à C₈, alcényle en C₂ à C₈ ou alcynyle en C₂ à C₈ pouvant être facultativement substitué avec un groupe COOH, OH, CO(CH₂)ₙCH₃, CO(CH₂)ₙCH₂N(R₆)₂ ou halogéno ; ou bien Z₁ et Z₂, conjointement, peuvent représenter un groupe -O-A-O- sur des atomes de carbone contigus ;
Z₃ représente un groupe Z₁ ou -X-R₉-Y ;
q est égal à zéro, un ou deux ;
n représente un nombre entier de 0 à six ;
m est égal à 1, 2 ou 3 ; et la ligne discontinue indique la présence facultative d'une double liaison ; ou un de ses sels pharmaceutiquement acceptables ; sous réserve que
. R₂ ne représente pas l'hydrogène lorsque X représente un groupe S(O)_{q} ;
. R₁₁ et R₇ ne représentent pas l'hydrogène lorsque q est égal à 1 ou 2 dans S(O)_{q}R₁₁ ou S(O)_{q}R₇ ;
. lorsque la double liaison facultative est présente, il existe un seul groupe R₁₀ et il n'existe aucun groupe P₁ et P₂ ne représente pas un groupe NR₆R₉Y ;
. lorsque la double liaison facultative est présente dans la formule (I) et X-R₂ est fixé à la double liaison, X'ne représente pas un groupe NR₆ ;
. lorsque la double liaison facultative est présente et R₁ est fixé directement à la double liaison, R₁ ne représente pas un groupe NR₆Ar ;
. lorsque R₃, R₅, Z₁, Z₂ ou Z₃ représente un groupe X(CH₂)ₙR₈ et n n'est pas égal à 0, X représente l'oxygène ou un groupe NR₆ lorsque R₈ représente un groupe OR₆ ou CO₂H ;
. lorsque R₈ représente un groupe CO₂C(R₁₁)₂O(CO)XR₇, X ne représente pas un groupe S(O)_{q} ;
. le composé de formule I ne consiste pas en le 1,3-diphénylindène-2-carboxylate de méthyle ; le 1,3-diphénylindène-2-carboxylate d'éthyle ; le 1,3-diphényl-2-cyano-indène ou le 1,3-diphényl-3-hydroxyindane-2-carboxylate d'éthyle ;
et sous réserve en outre que les composés dans lesquels :
R₁ représente un groupe -X(CH₂)ₙAr ou -X(CH₂)ₙR₈ ou 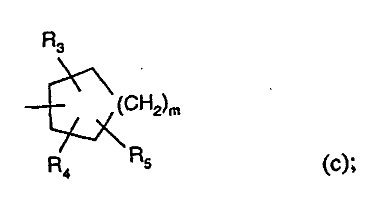
R₂ représente l'hydrogène, un groupe Ar ou (c) ;
P₁ représente un groupe -X(CH₂)R₈ ;
P₂ représente un groupe -X(CH₂)ₙR₈ ou -XR₉-Y ;
R₃ et R₅ représentent, indépendamment, l'hydrogène, un groupe R₁₁, OH, alkoxy en C₁ à C₈, S(O)_{q}R₁₁, N(R₆)₂, Br, F, I, Cl, CF₃, NHCOR₆, -XR₉-Y ou -X(CH₂)ₙR₈, les groupes méthylène de -X(CH₂)ₙR₈ pouvant être non substitués ou substitués avec un ou plusieurs groupes -(CH₂)ₙAr ;
R₄ représente l'hydrogène, un groupe R₁₁, OH, alkoxy en C₁ à C₅, S(O)_{q}R₁₁, N(R₆)₂, -X(R₁₁), Br, F, I, Cl ou NCOR₆, le groupe alkoxy en C₁ à C₅ pouvant être non substitué ou substitué avec un groupe OH, méthoxy ou halogéno ;
R₆ représente, indépendamment, l'hydrogène ou un groupe alkyle en C₁ à C₄ ;
R₇ représente, indépendamment, l'hydrogène, un groupe alkyle en C₁ à C₆ ou (CH₂)ₙAr ;
R₈ représente l'hydrogène, un groupe R₁₁, CO₂H, PO₃H₂, P(O) (OH)R₇ ou tétrazole ;
R₉ représente un groupe alkyle en C₁ à C₁₀, alcényle en C₂ à C₁₀ ou phényle, tous ces groupes pouvant être non substitués ou substitués avec un ou plusieurs groupes OH, N(R₆)₂, COOH, halogéno ou X(alkyle en C₁ à C₅) ;
R₁₀ représente un groupe R₃ ou R₄ ;
R₁₁ représente un groupe alkyle en C₁ à C₈, alcényle en C₂ à C₈ ou alcynyle en C₂ à C₈, tous ces groupes pouvant être non substitués ou substitués avec un ou plusieurs groupes OH, CH₂OH, N(R₆)₂ ou halogéno ;
X représente un groupe (CH₂)ₙ, O, NR₆ ou S(O)_{q} ;
Y représente un groupe CH₃ ou CH₂X(CH₂)ₙAr ;
Ar représente un groupe : 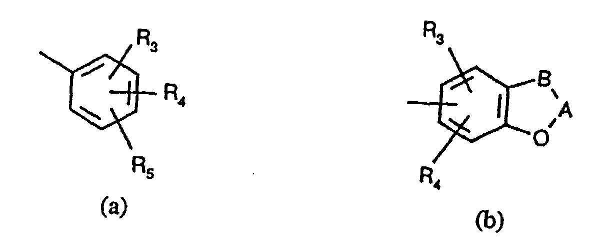
naphtyle, indolyle, pyridyle ou thiényle, oxazolidinyle, oxazolyle, thiazolyle, isothiazolyle, pyrazolyle, triazolyle, tétrazolyle, imidazolyle, imidazolidinyle, thiazolidinyle, isoxazolyle, oxadiazolyle, thiadiazolyle, morpholinyle, pipéridinyle, pipérazinyle, pyrrolyle ou pyrimidyle ; tous ces groupes pouvant être non substitués ou substitués avec un ou plusieurs groupes R₃ ou R₄ ;
A représente un groupe C=O ou [C(R₆)₂]ₘ ;
B représente un groupe -CH₂- ou -O- ;
Z₁ et Z₂ représentent, indépendamment, l'hydrogène, un groupe alkyle en C₁ à C₈, alcényle en C₂ à C₈, alcynyle en C₂ à C₈, OH, alkoxy en C₁ à C₈, S(O)_{q}(alkyle en C₁ à C₈), N(R₆)₂, Br, F, I, Cl, NHCOR₆, -X(CH₂)ₙR₈, phényle, benzyle ou cycloalkyle en C₃ à C₆, ledit groupe alkyle en C₁ à C₈, alcényle en C₂ à C₈ ou alcynyle en C₂ à C₈ pouvant être facultativement substitué avec un groupe COOH, OH, CO(CH₂)ₙCH₃, CO(CH₂)ₙCH₂N(R₆)₂ ou halogéno ; ou bien Z₁ et Z₂, conjointement, peuvent représenter un groupe -O-A-O- sur des atomes de carbone contigus ;
Z₃ représente un groupe Z₁ ou XR₉-Y ;
q est égal à zéro, un ou deux ;
n représente un nombre entier de 0 à six ; et
m est égal à 1, 2 ou 3 ; soient exclus.

2. Composé suivant la revendication 1, dans lequel :
R₁ représente un groupe X(CH₂)ₙAr, dihydrobenzofurannyle, benzodioxannyle, cyclohexyle ou alkyle en C₁ à C₄ ;
R₂ représente un groupement de formule (a) ou (b), alkyle en C₁ à C₄, indolyle ou l'hydrogène ;
R₃ et R₅ représentent, indépendamment, l'hydrogène, un groupe OH, alkoxy en C₁ à C₅, halogéno, -O(alkyle en C₁ à C₄)phényle, R₁₁CO₂R₇, alkyle en C₁ à C₄, N(R₆)₂, NH(CO)CH₃, -X(CH₂)ₙR₈, -X-R₉-Y, pyridyle, phényle ou S(O)_{q}(alkyle en C₁ à C₅) ;
R₄ représente l'hydrogène, un groupe OH, alkoxy en C₁ à C₅, halogéno, alkyle en C₁ à C₄, N(R₆)₂, NH(CO)CH₃ ou S(O)_{q}(alkyle en C₁ à C₅) ;
Z₁, Z₂ et Z₃ représentent, indépendamment, un groupe X-R₉-Y, benzyle, l'hydrogène, un groupe OH, alkoxy en C₁ à C₅, -N(R₆)₂, S(O)_{q}(alkyle en C₁ à C₈), NHCOR₆, X(CH₂)ₙR₈ ou halogéno, ou bien Z₁ et Z₂, conjointement, peuvent représenter un groupe -O-A-O- sur des atomes de carbone contigus ;
P₁ et P₂ représentent, indépendamment, l'hydrogène, un groupe CO₂H ou tétrazole ;
Ar représente un groupement de formule (a) ou (b) ou un groupe pyridyle ; et
X représente un groupe (CH₂)ₙ ou l'oxygène.

3. Composé suivant la revendication 1 ou 2, dans lequel :
R₃ représente l'hydrogène, un groupe -X(CH₂)ₙR₈ ou R₁₁CO₂R₇ ;
R₄ et R₅ représentent, indépendamment, l'hydrogène, un groupe OH, alkoxy en C₁ à C₅, S(alkyle en C₁ à C₅), F, Br, alkyle en C₁ à C₃ ou NH₂ ; et
Z₁ et Z₃ représentent l'hydrogène et Z₂ représente l'hydrogène, un groupe OH, alkoxy en C₁ à C₅, halogéno, X(CH₂)ₙR₈, NH₂, benzyle ou NH(CO)CH₃, ou bien Z₁ et Z₂, conjointement, peuvent représenter un groupe -O-A-O- sur des atomes de carbone contigus.

4. Composé suivant la revendication 1, 2 ou 3, dans lequel
R₁ représente un groupement de formule (b) ;
A représente un groupe CH₂, B représente -O- ;
il n'existe aucune double liaison facultative ;
R₁ et XR₂ sont en positions trans par rapport à P₁ ;
Z₂ représente un groupe OH, alkoxy en C₁ à C₅, -OCH₂CH₂CH₃ ou l'hydrogène ;
Z₁ représente l'hydrogène ;
R₃ repréente l'hydrogène, un groupe XAr, X(CH₂)_{q}CO₂H, X(CH₂)_{q}CONR₇SO₂R₁₁ ou CH=CHCO₂H ;
R₄ représente l'hydrogène ou un groupe alkoxy en C₁ ou C₂ ; et
R₅, R₁₀ et P₂ représentent l'hydrogène.

5. Composé qui consiste en l'acide (+) (1S,2R,3S)-3-(2-carboxyméthoxy-4-méthoxyphényl)-1-(3,4-méthylènedioxyphényl)-5-(prop-1-yloxy)indane-2-carboxylique ou un de ses sels pharmaceutiquement acceptables.

6. Composé qui consiste en l'acide (+) (1S,2R,3S)-3-[2-(2-hydroxyéth-1-yloxy)-4-méthoxyphényl]-1-(3,4-méthylènedioxyphényl)-5-(prop-1-yloxy)indane-2-carboxylique ou un de ses sels pharmaceutiquement acceptables.

7. Composé qui consiste en le sel disodique d'acide (+) (1S,2R,3S)-3-[2-(2-hydroxyéth-1-yloxy)-4-méthoxyphényl]-1-(3,4-méthylène-dioxyphényl)-5-(prop-1-yloxy)indane-2-carboxylique ou un de ses sels pharmaceutiquement acceptables.

8. Composé qui consiste en le sel de dicyclohexylamine d'acide (+) (1S,2R,3S)-3-[2-(2-hydroxyéth-1-yloxy)-4-méthoxyphényl]-1(3,4-méthylènedioxyphényl)-5-(prop-1-yloxy)indane-2-carboxylique.

9. Composé qui consiste en l'acide (1R,2SR,3RS)-3-[2-[(4-carboxypyridine-3-yl)oxy]-4-méthoxyphényl]-1-(3,4-méthylènedioxyphényl) -5-(prop-1-yl-oxy)indane-2-carboxylique ou un de ses sels pharmaceutiquement acceptables.

10. Composition pharmaceutique comprenant un composé suivant l'une quelconque des revendications 1 à 9 ou un de ses sels pharmaceutiquement acceptables et un support pharmaceutiquement acceptable.

11. Utilisation d'un composé suivant l'une quelconque des revendications 1 à 9 ou d'un de ses sels pharmaceutiquement acceptables dans la production d'un médicament destiné au traitement d'une affection nécessitant l'antagonisation des récepteurs d'endothéline.

12. Utilisation d'un composé suivant l'une quelconque des revendications 1 à 9 ou d'un des sels pharmaceutiquement acceptables dans la production d'un médicament destiné au traitement de l'hypertension, de l'insuffisance rénale ou d'une maladie vasculaire cérébrale.

13. Procédé pour la préparation d'un composé de formule (I) répondant à la définition suivant l'une quelconque des revendications 1 à 9 ou d'un de ses sels pharmaceutiquement acceptables, procédé qui comprend la réaction d'un composé de formule (11) : 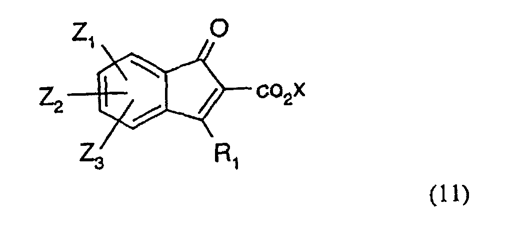 dans laquelle Z₁, Z₂, Z₃ et R₁ sont des groupes répondant aux définitions suivant la revendication 1 ou des groupes pouvant être convertis en ceux-ci, et X représente un groupe alkyle en C₁ à C₅, avec un composé organique de magnésium de formule (12) :
R₂(CH₂)ₙ-MgBr (12)
dans laquelle R₂ représente un groupe défini dans la revendication 1 ou un groupe pouvant être converti en celui-ci, dans un solvant convenable, ce qui donne un composé de formule (13) : 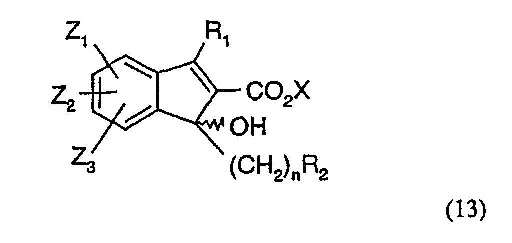 qui est réduit et ensuite, lorsque cela est désiré ou nécessaire, subit :
a) une insertion de R₁₀ (lorsqu'il est autre que l'hydrogène) par addition de conjugué ; et/ou
b) une alkylation ou acylation, ce qui donne des composés dans lesquels P₁ et P₂ sont autres que des groupes CO₂H ; et/ou
c) une conversion des groupes R₁, R₂, Z₁, Z₂ et Z₃ en d'autres groupes R₁, R₂, Z₁, Z₂ et Z₃ ; ce qui donne un composé de formule (I).
